(19) Europäisches Patentamt
European Patent Office
Office européen des brevets

(11) **EP 2 067 486 A1**

(12) **EUROPEAN PATENT APPLICATION**

(43) Date of publication:
**10.06.2009 Bulletin 2009/24**

(51) Int Cl.:
*A61K 45/00* (2006.01)    *A61K 45/06* (2006.01)
*A61K 39/395* (2006.01)    *A61P 35/00* (2006.01)
*A61P 35/02* (2006.01)    *A61K 31/70* (2006.01)

(21) Application number: **09157006.9**

(22) Date of filing: **31.01.2002**

(84) Designated Contracting States:
**AT BE CH CY DE DK ES FI FR GB GR IE IT LI LU MC NL PT SE TR**

(30) Priority: **31.01.2001  US 772938**
**16.05.2001  US 855717**
**05.11.2001  US 985646**

(62) Document number(s) of the earlier application(s) in accordance with Art. 76 EPC:
**02704280.3 / 1 370 292**

(71) Applicant: **Biogen Idec Inc.**
**San Diego, CA 92122 (US)**

(72) Inventors:
• **Hariharan,, Kandasamy**
**San Diego, CA 92129 (US)**

• **Hanna, Nabil**
**Rancho Santa Fe, CA 92067 (US)**
• **Braslawsky, Gary, R.**
**San Diego, CA 92129 (US)**
• **Pathan, Nuzhat**
**San Diego, CA 92129 (US)**

(74) Representative: **Adams, Harvey Vaughan John et al**
**Mathys & Squire LLP**
**120 Holborn**
**London**
**EC1N 2SQ (GB)**

Remarks:
This application was filed on 31-03-2009 as a divisional application to the application mentioned under INID code 62.

(54) **Use of CD23 antagonists for the treatment of neoplastic disorders**

(57)    Methods and kits for the treatment of neoplastic disorders comprising the use of a CD23 antagonist are provided. The CD23 antagonist may be used alone or in combination with chemotherapeutic agents. In particularly preferred embodiments the CD23 antagonists may be used to treat B cell chronic lymphocytic leukemia (B-CLL).

Fig. 1

**EP 2 067 486 A1**

**Description**

Cross Reference to Related Applications:

This application claims priority from US Serial No. 09/772,938 filed January 31, 2001, US Serial No. 09/855,717 filed May 16, 2001 and US Serial No. 09/985,646 filed November 5, 2001, each of which is incorporated in its entirety herein by reference.

Field of the Invention:

In a broad aspect the present invention relates to the use of CD23 antagonists for the treatment of neoplastic disorders. In preferred embodiments the present invention provides for the use of anti-CD23 antibodies for the immunotherapeutic treatment of malignancies including B cell chronic lymphocytic leukemia (B-CLL).

Background of the Invention:

Patients afflicted with relatively diverse malignancies have benefited from advances in cancer treatments over the past several decades. Unfortunately, while modern therapies have substantially increased remission rates and extended survival times, most patients continue to succumb to their disease eventually. Barriers to achieving even more impressive results comprise tumor-cell resistance and the unacceptable toxicity (e.g. myelotoxicity) of available treatments that limit optimal cytotoxic dosing and often make current therapies unavailable to immunocompromised, debilitated or older patients. These limitations are particularly evident when attempting to care for patients that have undergone previous treatments or have relapsed. Thus, it remains a challenge to develop less toxic, but more effective, targeted therapies.

One attempt at enhancing the effectiveness of such treatments involves the use of therapeutic antibodies to reduce undesirable cross-reactivity and increase tumor cell localization of one or more cytotoxic agents. The idea of recruiting antibodies to use in treating neoplastic disorders dates to at least 1953 when it was shown that antibodies could be used to specifically target tumor cells. However, it was the seminal work of Kohler and Milstein in hybridoma technology that allowed for a continuous supply of monoclonal antibodies that specifically target a defined antigen. By 1979, monoclonal antibodies (MAbs) had been used to treat malignant disorders in human patients. More recently two unconjugated monoclonal antibodies, Rituxan® & Herceptin®, have been approved for the treatment of non-Hodgkins lymphoma and breast cancer respectively. Currently, a number of monoclonal antibodies conjugated to cytotoxic agents (e.g. radioisotopes or protein toxins) are in clinical trials related to the treatment of various malignant disorders. Over the past decade, a wide variety of tumor-specific antibodies and antibody fragments have been developed, as have methods to conjugate the antibodies to drugs, toxins, radionuclides or other agents, and to administer the conjugates to patients. These efforts have produced great progress, but a variety of largely unanticipated problems have limited the diagnostic and therapeutic utility of some of the reagents thus far developed.

For example, among the most intractable problems is that which is caused by the human immune system itself. In many cases the patient's immune system responds to the targeting conjugate or therapeutic antibody as a foreign antigen. This is evidenced by patients treated with drugs or radionuclides complexed with murine monoclonal antibodies (which have been the most commonly used targeting antibodies for human) that develop circulating human anti-mouse antibodies (HAMAs) and a generalized immediate type-III hypersensitivity reaction to the antibody moiety of the conjugate. Furthermore, even when adverse side effects are minimal (for example, as in a single administration), circulating HAMAs decrease the effective concentration of the targeting agent in the patient and therefore limiting the diagnostic or therapeutic agent from reaching the target site.

One set of particularly attractive targets for directed immunotherapy are the hematological malignancies. Hematologic malignancies include lymphomas and leukemias that, in many instances , are more accessible to blood borne chemotherapeutics such as monoclonal antibodies than other types of tumors. While Rituxan has been shown to be effective in the treatment of some of these type of malignancies (particularly non-Hodgkins' lymphoma), there remain a number of hematologic malignancies for which there is no commonly accepted effective treatment. Among these malignancies is chronic lymphocytic leukemia.

Chronic lymphocytic leukemia (CLL or B-CLL) is predominantly a disease of the elderly that starts to increase in incidence after fifty years of age and reaches a peak by late sixties. It generally involves the proliferation of neoplastic peripheral blood lymphocytes. In the U.S.A., it has been estimated that in 1998, 7300 new cases of CLL were diagnosed and 4900 patients died of this disease accounting for 30% of leukemias in Western countries (Young and Percy et al. 1981 NIH Monograph 57; Cancer Facts and Figures, 1988, American Cancer Society Publication, Atlanta GA). Clinical finding of CLL involves lymphocytosis, lymphadenopathy, splenomegaly, anemia and thrombocytopenia. A characteristic feature of CLL is monoclonal B cell proliferation and accumulation of B-lymphocytes arrested at an intermediate state of differentiation (Dighiero and Travade et al, 1991, Blood 78:1901; Gale and Foon. 1985. Ann. Intern. Med 103:101). Such B cells express surface IgM (sIgM) or both sIgM and sIgD, and a single light chain at densities lower than that on the normal B cells. CLL B cells also display several human leukocyte antigens, including CD5, CD19, CD20, CD21, CD23, CD38 and CD64 (Foon and Todd, 1986. Blood 1:1). While the anti-CD20 antibody Rituxan has been used with some success, analysis of CLL patients shows that CD20 antigen density on CLL B cell to be highly variable with some patient's B cells expressing very low levels of CD20 antigen. Conversely, CD23 expression has been found to be consistently present at higher levels in B-CLL.

The CD23 leukocyte differentiation antigen is a 45 kD type II transmembrane glycoprotein expressed on several hematopoietic lineage cells, which function as a low affinity receptor for IgE (Fc$\gamma$RII) (Spiegelberg, 1984. Adv. Immunol. 35:61; Kikulani and Suemura et al 1986. J. Exp. Med 164;1455; Delespesse and Suter et al 1991. Adv. Immunol. 49: 149; Delespesse and Sarfati et al 1992. Immunol. Rev.125:77). It is a member of the C-type lectin family and contains an $\alpha$-helical coiled-coil stalk between the extracellular lectin binding domain and the transmembrane region. The stalk structure is believed to contribute to the oligomerization of membrane-bound CD23 to trimer during binding to its ligand (for example, IgE). Upon proteolysis, the membrane bound CD23 gives rise to several soluble CD23 (sCD23) molecular weight species (37 kD, 29 kD and 16 kD). Circulating sCD23 have been found in a range of clinical conditions at low serum concentrations ($\leq 5$ ng/ml), including CLL, rheumatoid arthritis and allergy. In CLL, sCD23 levels in serum correlated with the tumor burden and thus with the clinical stage of the disease (Sarfati and Bron et al. 1988. Blood 71:94). The sCD23, particularly the 25 kD species, has been shown to: a) act as an autocrine factor in some Fpstein-Barr virus transformed mature B-cell lines, b) act as a differentiation factor for prothymocytes and c) prevent apoptosis (programmed cell death) of germinal center B cells, possibly via the induction of *bcl-2* expression.

Besides Rituxan typical treatment for B-cell malignancies is the administration of radiation therapy and chemotherapeutic agents. In the case of CLL, conventional external radiation therapy will be used to destroy malignant cells. However, side effects are a limiting factor in this treatment. Another widely used treatment for hematological malignancies is chemotherapy. Combination chemotherapy has some success in reaching partial or complete remission. Unfortunately, these remissions obtained through chemotherapy are often not durable.

As such, it is an object of the present invention to provide low toxicity compounds and methods that may be used to target neoplastic cells.

It is another object of the invention to provide compounds and methods that may effectively used to treat neoplastic disorders and especially chronic lymphocytic leukemia in patients in need thereof.

Summary of the Invention:

These and other objectives are provided for by the present invention which, in a broad sense, is directed to methods, articles of manufacture, compounds and compositions that may be used in the treatment of neoplastic disorders. To that end, the present invention provides for CD23 antagonists that may be used to treat patients suffering from a variety of cancers. Thus in one aspect the present invention provides a method of treating a neoplastic disorder in a mammal in need thereof comprising administering a therapeutically effective amount of a CD23 antagonist to said mammal. As will be discussed in more detail below, CD23 antagonists may comprise any ligand, polypeptide, peptide, antibody or small molecule that interacts, binds or associates with the CD23 antigen expressed on B-cells and eliminates, reduces, inhibits or controls the growth of neoplastic cells. In preferred embodiments the CD23 antagonists of the instant invention will comprise anti-CD23 antibodies such as IDEC-152. In this respect it has been unexpectedly found that such antagonists may be used to induce apoptosis in neoplastic cells. Accordingly, another aspect of the instant invention comprises a method of inducing apoptosis in malignant cells comprising contacting said malignant cells with an apoptosis inducing amount of a CD23 antagonist. Moreover, as discussed in some detail below the CD23 antagonists may be used in an unconjugated state or conjugated with cytotoxic agents such as radioisotopes.

While the CD23 antagonists are effective anti-neoplastic agents in and of themselves, they may also be used synergistically in conjunction with various chemotherapeutic agents. Thus, another facet of the invention comprises a method of treating a neoplastic disorder in a mammal comprising the steps of:

administering a therapeutically effective amount of at least one chemotherapeutic agent to said mammal; and administering a therapeutically effective amount of at least one CD23 antagonist to said patient wherein said chemotherapeutic agent and said CD23 antagonist may be administered in any order or concurrently.

Although the CD23 antagonists may be used in conjunction with a number of chemotherapeutic agents, preferred embodiments of the invention comprise the use of the selected CD23 antagonist with the anti-CD20 antibody Rituxan®. In this respect yet another aspect of the invention comprises a method of treating a neoplastic disorder in a mammal comprising the steps of:

administering a therapeutically effective amount of Rituxan to said mammal; and
administering a therapeutically elective amount of IDEC-152 to said mammal wherein said Rittuxan and said IDEC-152 may be administered in any order or concurrently.

More generally, the antagonists of the instant invention may advantageously be used in combination with antibodies that react with or bind to antigens that are associated with malignancies for the treatment of neoplastic disorders. These include, but are not limited to CD19, CD20, CD22, CD40, CD40L, CD52 and B7 antigens.

Those skilled in the art will appreciate that the present invention may be used to treat any one of a number of CD23+ malignancies. As used herein a CD23+ malignancy is any neoplasm wherein the neoplastic cells express or are associated with the CD23 antigen. Exemplary CD23+ neoplasms that may be treated in accordance with the present invention comprise relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphoma, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas. Still other neoplasms that may be treated with the compositions of the instant invention comprise T cell lymphomas, acute T cell leukemias and mastocytomas.

While the methods of the present invention can be used to treat a number of CD23+ malignancies, it has been unexpectedly found that they are particularly effective in treating B cell chronic lymphocytic leukemia (B-CLL). As such one important aspect of the present invention comprises a method of treating B cell chronic lymphocytic leukemia (B-CLL) in a mammal in need thereof comprising administering a therapeutically effective amount of a CD23 antagonist to said mammal.

Yet another significant facet of the instant invention comprises articles of manufacture such as kits incorporating the disclosed CD23 antagonists. In this respect the present invention comprises a kit useful for the treatment of a mammal suffering from or predisposed to a neoplastic disorder comprising at least one container having a CD23 antagonist deposited therein and a label or an insert indicating that said CD23 antagonist may be used to treat said neoplastic disorder.

Other objects, features and advantages of the present invention will be apparent to those skilled in the art from a consideration of the following detailed description of preferred exemplary embodiments thereof.

Brief Description of the Figures:

Fig. 1 is a graphical representation of the specific binding of Rituxan® and a CD23 antagonist to lymphoma cells in a concentration dependent fashion;

Fig. 2 is a graphical representation of the antibody dependent cellular cytoxicity (ADCC) activity of a CD23 antagonist and Rituxan on lymphoma cells;

Figs. 3A and 3B show, respectively, the effects of the combination of a CD23 antagonist with Rituxan on ADCC mediated *in vitro* killing of tumor cells with low concentrations of the antagonist and with high concentrations of die antagonist;

Figs. 4A and 4B show, respectively, that the induction of apoptosis by a CD23 antagonist alone and with Rituxan in lymphoma cells;

Fig. 5 illustrates the induction of apoptosis by a CD23 antagonist and Rituxan with cross-linking by a secondary anti-human IgG specific antibody in lymphoma cells;

Figs. 6A and 6B illustrate, respectively, the induction of apoptosis by a CD23 antagonist and Rituxan and the combination thereof in SKW lymphoma cells after cross-linking with an anti-human IgG specific antibody;

Fig. 7 shows, the induction of apoptosis in lymphoma cells by a CD23 antagonist and Rituxan and the combination thereof at various concentrations;

Fig. 8 graphically illustrates the synergistic induction of apoptosis in lymphoma cells by a combination of a CD23 antagonist and Adriamycin;

Fig. 9 shows the synergistic induction of apoptosis in lymphoma cells by a combination of a CD23 antagonist and fludarabine;

Figs. 10A and 10B illustrate, respectively, the induction of apoptosis in CLL cells by a CD23 antagonist alone and the induction of apoptosis in CLL cells by a CD23 antagonist and Rituxan and the combination thereof at various concentrations;

Fig. 11 shows the induction of apoptosis in CLL cells by a combination of a CD23 antagonist and fludarabine;

Fig. 12 shows the anti-tumor activity of a CD23 antagonist as a single agent in a lymphoma/SCID mouse model;
Fig. 13 graphically illustrates the anti-tumor activity of a CD23 antagonist in combination with Rituxan in a lymphoma/SCID mouse model.

Detailed Description of the Invention:

While the present invention may be embodied in many different forms, disclosed herein are specific illustrative embodiments thereof that exemplify the principles of the invention. It should be emphasized that the present invention is not limited to the specific embodiments illustrated.

As indicated above, the present invention is directed to the use of CD23 antagonists for the treatment and or prophylaxis of any one of a number of CD23$^+$ malignancies. The disclosed antagonists may be used alone or in conjunction with a wide variety of chemotherapeutic agents. In this respect it has been unexpectedly discovered that the antagonists of the instant invention are particularly effective when used in conjunction with Rituxan. It has also been unexpectedly discovered that that CD23 antagonists of the present invention are especially efficacious in the treatment of chronic lymphocytic leukemia. Moreover, while not intending to limit the scope of the invention in any way, it has also been discovered that the CD23 antagonists disclosed herein can effectively induce apoptosis in neoplastic cells. This heretofore unknown property of the CD23 antagonists may be exploited according to the teachings herein to provide for therapeutically effective compositions.

In accordance with the present invention CD23 antagonists may comprise any ligand, polypeptide, peptide, antibody or small molecule that reacts, interacts, binds or associates with the CD23 antigen expressed on B-cells and eliminates, reduces, inhibits or controls the growth of neoplastic cells. As is known in the art CD23 refers to the low affinity receptor for IgE expressed by B cells and other cells. More particularly, a CD23 antagonist is a molecule which, upon binding to the CD23 cell surface marker, destroys or depletes CD23$^+$ cells in a mammal and/or interferes with one or more cell functions, *e.g.* by reducing or preventing a humoral response elicited by the cell.

The antagonist preferably is able to deplete B cells (*i.e.* reduce circulating B cell levels) in a mammal treated therewith. Such depletion may be achieved via various mechanisms such antibody-dependent cell-mediated cytotoxicity (ADCC), apoptosis and/or complement dependent cytotoxicity (CDC), inhibition of B' cell proliferation and/or induction of B cell death (*e.g.* via apoptosis). Antagonists included within the scope of the present invention include antibodies, synthetic or native sequence peptides and polypeptides, ligands and small molecule antagonists which bind to the CD23 cell marker, optionally conjugated with or fused to a cytotoxic agent.

Within the scope of the instant invention a particularly preferred CD23 antagonist is IDEC-152 (IDEC Pharmaceuticals, San Diego, California). IDEC-152 is a primatized monoclonal anti-CD23 antibody (also referred to herein as p5E8) against the CD23 antigen that has been developed for various indications (*Nakumura and Kloetzer et al. 2000. 22:131*). Monoclonal antibody p5E8 originated from 5E8, a primate anti-human CD23 antibody secreting hybridoma from cynomolgus macaques and was molecularly cloned and expressed as a 150 kDa IgG monomer in CHO cells using proprietary vector technology. Monoclonal p5E8 maintains the 5E8 primate variable region coupled to the human γ1 heavy chain and human k light chain constant regions. It also retains Clq binding. The sequence and derivation of IDEC-152 and other potential antagonists are disclosed in commonly owned U.S.P.N. 6,011,138 which is incorporated in its entirety herein by reference.

Those skilled in the art will appreciate that the compounds, compositions and methods of the present invention are useful for treating any neoplastic disorder, tumour or malignancy that exhibits CD23 (CD23$^+$ malignancies). As discussed above, the antagonists of the present invention are immunoreactive with CD23. In preferred embodiments where the antagonists are antibodies, they may be derived using common genetic engineering techniques whereby at least a portion of one or more constant region domains are deleted, substituted or altered so as to provide the desired biochemical characteristics such as reduced immunogenicity. It will further be appreciated that the antagonistic antibodies or immunoreactive fragments thereof may be expressed and produced on a clinical or commercial scale using well-established protocols.

For some embodiments it may be desirable to only use the antigen binding region (e.g., variable region or complementary determining regions) of the antagonistic antibody and combine them with a modified constant region to produce the desired properties. Compatible single chain constructs may be generated in a similar manner. In any event, it will be appreciated that the antibodies of the present invention may also be engineered to improve affinity or reduce immunogenicity as is common in the art. For example, CD23 antibodies compatible with the present invention may be derived or fabricated from antibodies that have been humanized or chimerized. Thus antibodies consistent with present invention may be derived from and/or comprise naturally occurring murine, primate (including human) or other mammalian monoclonal antibodies, chimeric antibodies, humanized antibodies, primatized antibodies, bispecific antibodies or single chain antibody constructs as well as immunoreactive fragments of each type.

The term "antibody" herein is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity.

"Antibody fragments" comprise a portion of an intact antibody, preferably comprising the antigen-binding or variable region thereof. Examples of antibody fragment include Fab, Fab', F(ab')Z, and Fv fragments; diabodies; linear antibodies; single-chain antibody molecules; and multispecific antibodies formed from antibody fragments. Domain deleted antibodies comprising immunoglobulins in which at least part of one or more constant regions have been altered or deleted to provide modified physiological properties (e.g. reduced serum half-life) may also be considered antibody fragments for the purposes of the instant disclosure. In preferred embodiments the domain deleted antibodies will comprise constant regions that lack the $C_H2$ domain.

Antagonists which "induce apoptosis" are those which induce programmed cell death, e.g. of a B cell, as determined by binding of annexin V, fragmentation of DNA, cell shrinkage, dilation of endoplasmic reticulum, cell fragmentation, and/or formation of membrane vesicles (called apoptotic bodies).

"Antibody-dependent cell-mediated cytotoxicity" and "ADCC" refer to a cell mediated reaction in which nonspecific cytotoxic cells that express Fc receptors (FcRs) *(e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be *assessed in vivo, e.g.*, in a animal model such as that disclosed in Clynes et al. PNAS (USA) 95:652-656 (1998).

As previously indicated, particularly preferred embodiments of the instant invention employ CD23 antagonists comprising antibodies to CD23 such as IDEC-152. While existing antibodies may be used in the instant invention new antibodies may be developed that are compatible with the disclosed methods. Using art recognized protocols, antibodies are preferably raised in mammals by multiple subcutaneous or intraperitoneal injections of the relevant antigen (e.g., purified tumor associated antigens or cells or cellular extracts comprising such antigens) and an adjuvant. This immunization typically elicits an immune response that comprises production of antigen-reactive antibodies from activated splenocytes or lymphocytes. While the resulting antibodies may be harvested from the serum of the animal to provide polyclonal preparations, it is often desirable to isolate individual lymphocytes from the spleen, lymph nodes or peripheral blood to provide homogenous preparations of monoclonal antibodies (MAbs). Preferably, the lymphocytes are obtained from the spleen.

In this well known process (Kohler et al., Nature, 256:495 (1975)) the relatively short-lived, or mortal, lymphocytes from a mammal which has been injected with antigen are fused with an immortal tumor cell line (e.g. a myeloma cell line), thus producing hybrid cells or "hybridomas" which are both immortal and capable of producing the genetically coded antibody of the B cell. The resulting hybrids are segregated into single genetic strains by selection, dilution, and regrowth with each individual strain comprising specific genes for the formation of a single antibody. They therefore produce antibodies which are homogeneous against a desired antigen and, in reference to their pure genetic parentage, are termed "monoclonal."

Hybridoma cells thus prepared are seeded and grown in a suitable culture medium that preferably contains one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. Preferably, the binding specificity of the monoclonal antibodies produced by hybridoma cells is determined by immunoprecipitation or by an *in vitro* assay, such as a radioimmunoassay (RIA) or enzyme-linked immunoabsorbent assay (ELISA). After hybridoma cells are identified that produce antibodies of the desired specificity, affinity and/or activity, the clones may be subcloned by limiting dilution procedures and grown by standard methods (Coding, Monoclonal Antibodies: Principles and Practice, pp 59-103 (Academic Press, 1986)). It will further be appreciated that the monoclonal antibodies secreted by the subclones may be separated from culture medium, ascites fluid or serum by conventional purification procedures such as, for example, protein-A, hydroxylapatite chromatography, gel electrophoresis, dialysis or affinity chromatography.

In other compatible embodiments, DNA encoding the desired monoclonal antibodies may be readily isolated and sequenced using conventional procedures (e.g., by using oligonucleotide probes that are capable of binding specifically to genes encoding the heavy and light chains of murine antibodies). The isolated and subcloned hybridoma cells serve as a preferred source of such DNA. Once isolated, the DNA may be placed into expression vectors, which are then transfected into prokaryotic or eukaryotic host cells such as *E. coli* cells, simian COS cells, Chinese Hamster Ovary (CHO) cells or myeloma cells that do not otherwise produce immunoglobulins. More particularly, the isolated DNA (which may be modified as described herein) may be used to clone constant and variable region sequences for the manufacture antibodies as described in Newman et al., U.S. Serial No. 379,072, filed January 25, 1995. which is incorporated by reference herein. Essentially, this entails extraction of RNA from the selected cells, conversion to cDNA, and amplification thereof by PCR using Ig specific primers. Suitable primers are described in U.S. Pat. No. 5,658,570 which is also incorporated herein by reference. As will be discussed in more detail below, transformed cells expressing the desired antibody may be grown up in relatively large quantities to provide clinical and commercial supplies of the immunoglobulin.

Those skilled in the art will also appreciate that DNA encoding antibodies or antibody fragments may also be derived from antibody phage libraries as set forth, for example, in EP 368 684 B1 and U.S.P.N. 5,969,108 each of which is incorporated herein by reference. Several publications (e.g., Marks et al. Bio/Technology 10:779-783 (1992)) have described the production of high affinity human antibodies by chain shuffling, as well as combinatorial infection and *in vivo* recombination as a strategy for constructing large phage libraries. Such procedures provide viable alternatives to traditional hybridoma techniques for the isolation and subsequent cloning of monoclonal antibodies and, as such, are clearly within the purview of the instant invention.

Yet other embodiments of the present invention comprise the generation of substantially human antibodies in transgenic animals (e.g., mice) that are incapable of endogenous immunoglobulin production (see e.g., U.S. Pat. Nos. 6,075,181, 5,939,598, 5,591,669 and 5,589,369 each of which is incorporated herein by reference). For example, it has been described that the homozygous deletion of the antibody heavy-chain joining region in chimeric and germ-line mutant mice results in complete inhibition of endogenous antibody production. Transfer of a human immunoglobulin gene array in such germ line mutant mice will result in the production of human antibodies upon antigen challenge. Another preferred means of generating human antibodies using SCID mice is disclosed in commonly-owned, co-pending U.S. Pat. No. 5,811,524 which is incorporated herein by reference. It will be appreciated that the genetic material associated with these human antibodies may also be isolated and manipulated as described herein.

Yet another highly efficient means for generating recombinant antibodies is disclosed by Newman, Biotechnology, 10: 1455-1460 (1992). Specifically, this technique results in the generation of primatized antibodies that contain monkey variable domains and human constant sequences. This reference is incorporated by reference in its entirety herein. Moreover, this technique is also described in commonly assigned U.S. Pat. Nos. 5,658,570, 5,693,780 and 5,756,096 each of which is incorporated herein by reference. One preferred embodiment of the instant invention, IDEC-152 was generated using the techniques as substantially described in the foregoing references.

As is apparent from the instant specification, genetic sequences useful for producing antibody derivatives of the CD23 antagonists of the present invention may be obtained from a number of different sources. For example, as discussed extensively above, a variety of human antibody genes are available in the form of publicly accessible deposits. Many sequences of antibodies and antibody-encoding genes have been published and suitable antibody genes can be synthesized from these sequences much as previously described. Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991) which is herein incorporated by reference in its entirety, including supplements.

It will further be appreciated that the scope of this invention further encompasses all alleles, variants and mutations of the DNA sequences described herein.

As is well known, RNA may be isolated from the original hybridoma cells or from other transformed cells by standard techniques, such as guanidinium isothiocyanate extraction and precipitation followed by centrifugation or chromatography. Where desirable, mRNA may be isolated from total RNA by standard techniques such as chromatography on oligodT cellulose. Techniques suitable to these purposes are familiar in the art and are described in the foregoing references.

cDNAs that encode the light and the heavy chains of the antibody may be made, either simultaneously or separately, using reverse transcriptase and DNA polymerase in accordance with well known methods. It may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as mouse constant region probes.

DNA, typically plasmid DNA, may be isolated from the cells as described herein, restriction mapped and sequenced in accordance with standard, well known techniques set forth in detail in the foregoing references relating to recombinant DNA techniques. Of course, the DNA may be modified according to the present invention at any point during the isolation process or subsequent analysis.

Preferred antibody sequences are disclosed herein. Oligonucleotide synthesis techniques compatible with this aspect of the invention are well known to the skilled artisan and may be carried out using any of several commercially available automated synthesizers. In addition, DNA sequences encoding several types of heavy and light chains set forth herein can be obtained through the services of commercial DNA synthesis vendors. The genetic material obtained using any of the foregoing methods may then be altered or modified to provide antibodies compatible with the present invention.

While a variety of different types of antibodies may be obtained and modified according to the instant invention, the modified antibodies of the instant invention will share various common traits. To that end, the term "immunoglobulin" shall be held to refer to a tetramer or aggregate thereof whether or not it possesses any relevant specific immunoreactivity. "Antibodies" refers to such assemblies which have significant known specific immunoreactive activity to an antigen (e.g. a tumor associated antigen), comprising light and heavy chains, with or without covalent linkage between them. The antibodies may be modified to provide beneficial physiological characteristics. The term "modified antibodies" according to the present invention are held to mean antibodies, or immunoreactive fragments or recombinants thereof, in which at least a fraction of one or more of the constant region domains has been deleted or otherwise altered so as to provide desired biochemical characteristics such as increased tumor localization or reduced serum half-life when compared with a whole, unaltered antibody of approximately the same immunogenicity. For the purposes of the instant application, immunoreactive single chain antibody constructs having altered or omitted constant region domains may be considered to be modified antibodies.

Basic immunoglobulin structures in vertebrate systems are relatively well understood. As will be discussed in more detail below, the generic term "immunoglobulin" comprises five distinct classes of antibody that can be distinguished biochemically. While all five classes are clearly within the scope of the present invention, the following discussion will generally be directed to the class of IgG molecules. With regard to IgG, immunoglobulins comprise two identical light polypeptide chains of molecular weight approximately 23,000 Daltons, and two identical heavy chains of molecular weight 53,000-70,000. The four chains are joined by disulfide bonds in a "Y" configuration wherein the light chains bracket the heavy chains starting at the mouth of the "Y" and continuing through the variable region.

More specifically, both the light and heavy chains are divided into regions of structural and functional homology. The terms "constant" and "variable" are used functionally. In this regard, it will be appreciated that the variable domains of both the light ($V_L$) and heavy ($V_H$) chains determine antigen recognition and specificity. Conversely, the constant domains of the light chain ($C_L$) and the heavy chain ($C_H1$, $C_H2$ or $C_H3$) confer important biological properties such as secretion, transplacental mobility, Fc receptor binding, complement binding, and the like. By convention the numbering of the constant region domains increases as they become more distal from the antigen binding site or amino-terminus of the antibody. Thus, the $C_H3$ and $C_L$ domains actually comprise the carboxy-terminus of the heavy and light chains respectively.

Light chains are classified as either kappa or lambda ($\kappa$, $\lambda$). Each heavy chain class may be bound with either a kappa or lambda light chain. In general, the light and heavy chains are covalently bonded to each other, and the "tail" portions of the two heavy chains are bonded to each other by covalent disulfide linkages when the immunoglobulins are generated either by hybridomas, B cells or genetically engineered host cells. However, if non-covalent association of the chains can be effected in the correct geometry, the aggregate of non-disulfide-linked chains will still be capable of reaction with antigen. In the heavy chain, the amino acid sequences run from an N-terminus at the forked ends of the Y configuration to the C-terminus at the bottom of each chain. At the N-terminus is a variable region and at the C-terminus is a constant region. Those skilled in the art will appreciate that heavy chains are classified as gamma, mu, alpha, delta, or epsilon, ($\gamma$, $\mu$, $\alpha$, $\delta$, $\varepsilon$) with some subclasses among them. It is the nature of this chain that determines the "class" of the antibody as IgA, IgD, IgE IgG, or IgM. The immunoglobulin subclasses (isotypes) e.g. $IgG_1$, $IgG_2$, $IgG_3$, $IgG_4$, $IgA_1$, etc. are well characterized and are known to center functional specialization. Modified versions of each of these classes and isotypes are readily discernable to the skilled artisan in view of the instant disclosure and, accordingly, are within the purview of the instant invention.

As indicated above, the variable region allows the antibody to selectively recognize and specifically bind epitopes on immunoreactive antigens. That is, the $V_L$ domain and $V_H$ domain of an antibody combine to form the variable region that defines a three dimensional antigen binding site. This quaternary antibody structure provides for an antigen binding site present at the end of each arm of the Y. More specifically, the antigen binding site is defined by three complementary determining regions (CDRs) on each of the $V_H$ and $V_L$, chains.

The six CDRs are short, non-contiguous sequences of amino acids that are specifically positioned to form the antigen binding site as the antibody assumes its three dimensional configuration in an aqueous environment. The remainder of the heavy and light variable domains show less inter-molecular variability in amino acid sequence and are termed the framework regions. The framework regions largely adopt a $\beta$-sheet conformation and the CDRs form loops connecting, and in some cases forming part of, the $\beta$-sheet structure. Thus, these framework regions act to form a scaffold that provides for positioning the six CDRs in correct orientation by inter-chain, non-covalent interactions. In any event, the antigen binding site formed by the positioned CDRs defines a surface complementary to the epitope on the immunoreactive antigen. This complementary surface promotes the non-covalent binding of the antibody to the immunoreactive antigen epitope.

For the purposes of the present invention, it should be appreciated that the disclosed anti-CD23 antibodies may comprise any type of variable region that provides for the association of the antibody with CD23 marker. In this regard, the variable region may comprise or be derived from any type of mammal that can be induced to mount a humoral response and generate immunoglobulins against CD23. As such, the variable region of the antagonistic antibodies may be, for example, of human, murine, non-human primate (e.g. cynomolgus monkeys, macaques, etc.) or lupine origin. In preferred embodiments both the variable and constant regions of the immunoglobulins are human. In other selected embodiments the variable regions of compatible antibodies (usually derived from a non-human source) may be engineered or specifically tailored to improve the binding properties or reduce the immunogenicity of the molecule. In this respect, variable regions useful in the present invention may be humanized or otherwise altered through the inclusion of imported amino acid sequences.

By "humanized antibody" is meant an antibody derived from a non-human antibody, typically a murine antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting at least a part of one or more of the non-human complementarity determining regions (CDRs) into human framework and constant regions with or without retention of critical framework residues; or (c) transplanting the entire non-human variable domains, but "cloaking" them with a human-like section by replacement of surface residues. Such methods are disclosed in Morrison et al., Proc. Natl. Acad. Sci. 81: 685.1-5 (1984); Morrison et al., Adv. Immunol. 44: 65-92 (1988); Verhoeyen et al., Science 239: 1534-1536 (1988); Padlan, Molec. Immun. 28: 489-498 (1991); Padlan, Molec. Immun. 31: 169-217 (1994), and U.S. Pat. Nos. 5.585,089, 5,693,761 and 5,693,762 all of which are hereby incorporated by reference in their entirety.

Those skilled in the art will appreciate that the technique set forth in option (a) above will produce "classic" chimeric antibodies. In the context of the present application the term "chimeric antibodies" will be held to mean any antibody wherein the immunoreactive region or site is obtained or derived from a first species and the constant region (which may be intact, partial or modified in accordance with the instant invention) is obtained from a second species. In preferred embodiments the antigen binding region or site will be from a non-human source (e.g. primate or mouse) and the constant region is human. While the immunogenic specificity of the variable region is not generally affected by its source, a human constant region is less likely to elicit an immune response from a human subject than would the constant region from a non-human source.

Preferably, the variable domains in both the heavy and light chains are altered by at least partial replacement of one or more CDRs and, if necessary, by partial framework region replacement and sequence changing. Although the CDRs may be derived from an antibody of the same class or even subclass as the antibody from which the framework regions are derived, it is envisaged that the CDRs will be derived from an antibody of different class and preferably from an antibody from a different species. It must be emphasized that it may not be necessary to replace all of the CDRs with the complete CDRs from the donor variable region to transfer the antigen binding capacity of one variable domain to another. Rather, it may only be necessary to transfer those residues that are necessary to maintain the activity of the antigen binding site. Given the explanations set forth in U. S. Pat. Nos. 5,585,089, 5,693,761 and 5,693,762, it will be well within the competence of those skilled in the art, either by carrying out routine experimentation or by trial and error testing to obtain a functional antibody with reduced immunogenicity.

Alterations to the variable region notwithstanding, those skilled in the art will appreciate that, in preferred embodiments, the anti-CD23 antibodies of the instant invention may comprise antibodies, or immunoreactive fragments thereof, in which at least a fraction of one or more of the constant region domains has been deleted or otherwise altered so as to provide desired biochemical characteristics such as increased tumor localization or reduced serum half-life when compared with an antibody of approximately the same immunogenicity comprising a native or unaltered constant region. In selected embodiments, the constant region of these type of anti-CD23 antibodies will comprise a human constant region. Modifications to the constant region compatible with the instant invention comprise additions, deletions or substitutions of one or more amino acids in one or more domains. That is, the anti-CD23 antibodies disclosed herein may comprise alterations or modfications to one or more of the three heavy chain constant domains ($C_H1$, $C_H2$ or $C_H3$) and/or to the light chain constant domain ($C_L$). In especially preferred embodiments the modified antibodies will comprise domain deleted constructs or variants wherein the entire $C_H2$ domain has been removed ($\Delta C_H2$ constructs).

Besides their configuration, it is known in the art that the constant region mediates several effector functions. For example, binding of the C1 component of complement to antibodies activates the complement system. Activation of complement is important in the opsonisation and lysis of cell pathogens. The activation of complement also stimulates the inflammatory response and may also be involved in autoimmune hypersensitivity. Further, antibodies bind to cells via the Fc region, with a Fc receptor site on the antibody Fc region binding to a Fc receptor (FcR) on a cell. There are a number of Fc receptors which are specific for different classes of antibody, including IgG (gamma receptors), IgE (eta receptors), IgA (alpha receptors) and IgM (mu receptors). Binding of antibody to Fc receptors on cell surfaces triggers a number of important and diverse biological responses including engulfment and destruction of antibody-coated particles, apoptosis, clearance of immune complexes, lysis of antibody-coated target cells by killer cells (called antibody-dependent cell-mediated cytotoxicity, or ADCC), release of inflammatory mediators, placental transfer and control of immunoglobulin production.

Following manipulation of the isolated genetic material to provide CD23 antagonists such as antibodies and reactive polypeptides as set forth above, the nucleic acids are typically inserted in an expression vector for introduction into host cells that may be used to produce the desired quantity of CD23 antagonist.

The term "vector" or "expression vector" is used herein for the purposes of the specification and claims, to mean vectors used in accordance with the present invention as a vehicle for introducing into and expressing a desired nucleic acid sequence in a cell. As known to those skilled in the art, such vectors may easily be selected from the group consisting of plasmids, phages, viruses and retroviruses. In general, vectors compatible with the instant invention will comprise a selection marker, appropriate restriction sites to facilitate cloning of the desired gene and the ability to enter and/or replicate in eukaryotic or prokaryotic cells.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Additionally, cells which have integrated the DNA into their chromosomes may be selected by introducing one or more markers which allow selection of transected host cells. The marker may provide for prototrophy to an auxotrophic host, biocide resistance (e.g., antibiotics) or resistance to heavy metals such as copper. The selectable marker gene can either be directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals.

In particularly preferred embodiments directed to anti-CD23 antibodies, the cloned variable region genes are inserted into an expression vector along with the heavy and light chain constant region genes (preferably human) as discussed above. Preferably, this is effected using a proprietary expression vector of IDEC Pharmaceuticals, Inc. (San Diego, California), referred to as NEOSPLA. This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. As seen in the examples below, this vector has been found to result in very high level expression of antibodies upon incorporation of variable and constant region genes, transfection in CHO cells, followed by selection in G418 containing medium and methotrexate amplification. This vector system is substantially disclosed in commonly assigned U.S. Pat. Nos. 5,736,137 and 5,658,570, each of which is incorporated by reference in its entirety herein. This system provides for high expression levels, *i.e.,* > 30 pg/cell/day. Reactive polypeptide antagonists may be expressed using similar vectors.

More generally, once the vector or DNA sequence containing the reactive polypeptide or antibody has been prepared, the expression vector may be introduced into an appropriate host cell. That is, the host cells may be transformed. Introduction of the plasmid into the host cell can be accomplished by various techniques well known to those of skill in the art. These include, but are not limited to, transfection (including electrophoresis and clectroporation), protoplast fusion, calcium phosphate precipitation, cell fusion with enveloped DNA, microinjection, and infection with intact virus. See, Ridgway, A. A. G. "Mammalian Expression Vectors" Chapter 24.2, pp. 470-472 Vectors, Rodriguez and Denhardt, Eds. (Butterworths, Boston, Mass. 1988). Most preferably, plasmid introduction into the host is via electroporation. The transformed cells are grown under conditions appropriate to the production of the light chains and heavy chains, and assayed for heavy and/or light chain protein synthesis. Exemplary assay techniques include enzyme-linked immmosorb-ent assay (ELISA), radioimmunoassay (RIA), or flourescence-activated cell sorter analysis (FACS), immunohistochemistry and the like.

As used herein, the term "transformation" shall be used in a broad sense to refer to any introduction of DNA into a recipient host cell that changes the genotype and consequently results in a change in the recipient cell.

Along those same lines, "host cells" refers to cells that have been transformed with vectors constructed using recombinant DNA techniques and containing at least one heterologous gene. As defined herein, the antibody or modification thereof produced by a host cell is by virtue of this transformation. In descriptions of processes for isolation of antibodies from recombinant hosts, the terms "cell" and "cell culture" are used interchangeably to denote the source of antibody unless it is clearly specified otherwise. In other words, recovery of antibody from the "cells" may mean either from spun down whole cells, or from the cell culture containing both the medium and the suspended cells.

The host cell line used for protein expression is most preferably of mammalian origin; those skilled in the art are credited with ability to preferentially determine particular host cell lines which are best suited for the desired gene product to be expressed therein. Exemplary host cell lines include, but are not limited to, DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3.times.63-Ag3.653 (mouse myeloma), BFA-1c1BPT (bovine endothelial cells), RAJI (human lymphocyte) and 293 (human kidney). CHO cells are particularly preferred. Host cell lines are typically available from commercial services, the American Tissue Culture Collection or from published literature.

*In vitro* production allows scale-up to give large amounts of the desired CD23 antagonists. Techniques for mammalian cell cultivation wider tissue culture conditions are known in the art and include homogeneous suspension culture, e.g. in an airlift reactor or in a continuous stirrer reactor, or immobilized or entrapped cell culture, e.g. in hollow fibers, microcapsules, on agarose microbeads or ceramic cartridges. For isolation of the CD23 antagonists, the expressed polypeptide in the culture supernatants are first concentrated, e.g. by precipitation with ammonium sulphate, dialysis against hygroscopic material such as PEG, filtration through selective membranes, or the like. If necessary and/or desired, the concentrated polypeptides are purified by the customary chromatography methods, for example gel filtration, ion-exchange chromatography, chromatography over DEAE-cellulose or (immuno-)affinity chromatography.

The reactive polypeptide genes can also be expressed non-mammalian cells such as bacteria or yeast. In this regard it will be appreciated that various unicellular non-mammalian microorganisms such as bacteria can also be transformed; i.e. those capable of being grown in cultures or fermentation. Bacteria, which are susceptible to transformation, include members of the enterobacteriaceae, such as strains of Escherichia coli; Salmonella; Bacillaceae, such as Bacillus subtilis; Pneumococcus; Streptococcus, and Haemophilus influenzae. It will further be appreciated that, when expressed in bacteria, anti-CD23 immunoglobulin heavy chains and light chains typically become part of inclusion bodies. The chains then must be isolated, purified and then assembled into functional immunoglobulin molecules.

In addition to prokaryates, eukaryotic microbes may also be used. Saccharomyces cerevisiae, or common baker's yeast, is the most commonly used among eukaryotic microorganisms although a number of other strains are commonly available.

For expression in Saccharomyces, the plasmid YRp7, for example, (Stinchcomb et al., Nature, 282:39 (1979); Kingsman et al., Gene, 7:141 (1979); Tschemper et al., Gene, 10:157 (1980)) is commonly used. This plasmid already contains the trpl gene which provides a selection marker for a mutant strain of yeast lacking the ability to grow in tryptophan, for example ATCC No. 44076 or PEP4-1 (Jones, Genetics, 85:12 (1977)). The presence of the trpl lesion as a characteristic of the yeast host cell genome then provides an effective environment for detecting transformation by growth in the absence of tryptophan.

Regardless of how clinically useful quantities are obtained, the CD23 antagonists of the present invention may be used in any one of a number of conjugated (i.e. an immunoconjugate) or unconjugated forms. In particular, the antibodies of the present invention may be conjugated to, or associated with cytotoxins such as radioisotopes, therapeutic agents, cytostatic agents, biological toxins or prodrugs. Alternatively, the CD23 antagonists of the instant invention may be used in a nonconjugated or "naked" form to harness the subject's natural defense mechanisms including complement-dependent cytotoxicity (CDC), antibody dependent cellular toxicity (ADCC) or apoptosis to eliminate the malignant cells. In particularly preferred embodiments, the CD23 antagonists may be conjugated to radioisotopes, such as $^{90}$Y, $^{125}$I, $^{131}$I, $^{123}$I, $^{111}$In, $^{105}$Rh, $^{153}$Sm, $^{67}$Cu, $^{67}$Ga, $^{166}$Ho, $^{177}$Lu, $^{186}$Re and $^{188}$Re using anyone of a number of well known chelators or direct labeling. In other embodiments, the disclosed compositions may comprise CD23 antagonists associated with drugs, prodrugs or biological response modifiers such as methotrexate, adriamycin, and lymphokines such as interferon. Still other embodiments of the present invention comprise the use of antibodies conjugated to specific biotoxins such as ricin or diptheria toxin. In yet other embodiments the CD23 antagonists may be complexed with other immunologically active ligands (e.g. antibodies or fragments thereof) wherein the resulting molecule binds to both the neoplastic cell and an effector cell such as a T cell. The selection of which conjugated or unconjugated CD23 antagonists to use will depend of the type and stage of cancer, use of adjunct treatment (e.g., chemotherapy or external radiation) and patient condition. It will be appreciated that one skilled in the art could readily make such a selection in view of the teachings herein.

As used herein, "a cytotoxin or cytotoxic agent" means any agent that may be associated with the disclosed CD23 antagonists that is detrimental to the growth and proliferation of cells and may act to reduce, inhibit or distroy a malignancy when exposed thereto. Exemplary cytotoxins include, but are not limited to, radionuclides, biotoxins, cytostatic or cytotoxic therapeutic agents, prodrugs, immunologically active ligands and biological response modifiers such as cytokines. As will be discussed in more detail below, radionuclide cytotoxins are particularly preferred for use in the instant invention. However, any cytotoxin that acts to retard or slow the growth of malignant cells or to eliminate malignant cells and may be associated with the CD23 antagonists disclosed herein is within the purview of the present invention.

It will be appreciated that, in previous studies, anti-tumor antibodies labeled with these isotopes have been used successfully to destroy cells in solid tumors as well as lymphomas/leukemias in animal models, and in some cases in humans. The radionuclides act by producing ionizing radiation which causes multiple strand breaks in nuclear DNA, leading to cell death. The isotopes used to produce therapeutic conjugates typically produce high energy $\alpha$- or $\beta$-particles which have a short path length. Such radionuclides kill cells to which they are in close proximity, for example neoplastic cells to which the conjugate has attached or has entered. They have little or no effect on non-localized cells. Radionuclides are essentially non-immunogenic.

With respect to the use of radiolabeled conjugates in conjunction with the present invention, the CD23 antagonists may be directly labeled (such as through iodination) or may be labeled indirectly through the use of a chelating agent. As used herein, the phrases "indirect labeling" and "indirect labeling approach" both mean that a chelating agent is covalently attached to an antibody and at least one radionuclide is associated with the chelating agent. Particularly preferred chelating agents are bifunctional chelating agents and comprise, for example, 1-isothiocycmatobenzyl-3-methyldiothelene triaminepentaacetic acid ("MX-DTPA") and cyclohexyl diethylenetriamine pentaacetic acid ("CHX-DTPA") derivatives. Particularly preferred radionuclides for indirect labeling include $^{111}$In and [90]Y.

As used herein, the phrases "direct labeling" and "direct labeling approach" both mean that a radionuclide is covalently attached directly to a CD23 antagonist (typically via an amino acid residue). More specifically, these linking technologies include random labeling and site-directed labeling. In the latter case, the labeling is directed at specific sites on the dimer or tetramer, such as the N-linked sugar residues present only on the Fc portion of the conjugates. Further, various direct labeling techniques and protocols are compatible with the instant invention. For example, Technetium-99m labelled antibodies may be prepared by ligand exchange processes, by reducing pertechnate ($TcO_4^-$) with stannous ion solution, chelating the reduced technetium onto a Sephadex column and applying the antibodies to this column, or by batch labelling techniques, e.g. by incubating pertechnate, a reducing agent such as $SnCl_2$, a buffer solution such as a sodium-potassium phthalate-solution, and the CD23 antagonists. In any event, preferred radionuclides for directly labeling CD23 antagonists are well known in the art and a particularly preferred radionuclide for direct labeling is $^{131}$I covalently attached via tyrosine residues. CD23 antagonists according to the invention may be derived, for example, with radioactive sodium or potassium iodide and a chemical oxidising agent, such as sodium hypochlorite, chloramine T or the like, or an enzymatic oxidising agent, such as lactoperoxidase, glucose oxidase and glucose. However, for the purposes of the present invention, the indirect labeling approach is particularly preferred.

Patents relating to chelators and chelator conjugates are known in the art. For instance, U.S. Patent No. 4,831,175 of Gansow is directed to polysubstituted diethylenetriaminepentaacetic acid chelates and protein conjugates containing the same, and methods for their preparation. U.S. Patent Nos. 5,099,069, 5,246,692, 5,286,850, 5,434,287 and 5,124,471 of Gansow also relate to polysubstituted DTPA chelates. These patents are incorporated herein in their entirety. Other examples of compatible metal chelators are ethylenediaminetetraacetic acid (EDTA), diethylenetriaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like. Other compatible chelates, including those yet to be discovered, may easily be discerned by a skilled artisan and are clearly within the scope of the present invention.

Compatible chelators, including the specific bifunctional chelator used to facilitate chelation in co-pending application Serial Nos. 08/475,813, 08/475,815 and 08/478,967, are preferably selected to provide high affinity for trivalent metals; exhibit increased tumor-to-non-tumor ratios and decreased bone uptake as well as greater *in vivo* retention of radionuclides at target sites, i.e., B-cell lymphoma tumor sites. However, other bifunctional chelator that may or may not possess all of these characteristics are known in the art and may also be beneficial in tumor therapy.

It will also be appreciated that, in accordance with the teachings herein, the CD23 antagonists may be conjugated to different radiolabels for diagnostic and therapeutic purposes. To this end the aforementioned co-pending applications, herein incorporated by reference in their entirety, disclose radiolabeled therapeutic conjugates for diagnostic "imaging" of tumors before administration of therapeutic antibody. $^{111}$In is particularly preferred as a diagnostic radionuclide because between about 1 to about 10 mCi can be safely administered without detectable toxicity; and the imaging data is generally predictive of subsequent $^{90}$Y-labeled antibody distribution. Most imaging studies utilize 5 mCi $^{111}$In-labeled antibody, because this dose is both safe and has increased imaging efficiency compared with lower doses, with optimal imaging occurring at three to six days after antibody administration. See, for example, Murray, J. Nuc. Med. 26: 3328 (1985) and Carraguillo et al., J. Nuc. Med. 26: 67 (1985).

As indicated above, a variety of radionuclides are applicable to the present invention and those skilled in the art are credited with the ability to readily determine which radionuclide is most appropriate under various circumstances. For example, $^{131}$I is a well known radionuclide used for targeted immunotherapy. However, the clinical usefulness of $^{131}$I can be limited by several factors including: eight-day physical half-life; dehalogenation of iodinated antibody both in the blood and at tumor sites; and emission characteristics (*e.g.*, large gamma component) which can be suboptimal for localized dose deposition in tumor. With the advent of superior chelating agents, the opportunity for attaching metal chelating groups to proteins has increased the opportunities to utilize other radionuclides such as $^{111}$In and [90]Y. $^{90}$Y provides several benefits for utilization in radioimmunotherapeutic applications: the 64 hour half-life of $^{90}$Y is long enough to allow antibody accumulation by tumor and, unlike *e.g.*, $^{131}$I $^{90}$Y is a pure beta emitter of high energy with no accompanying gamma irradiation in its decay, with a range in tissue of 100 to 1,000 cell diameters. Furthermore, the minimal amount of penetrating radiation allows for outpatient administration of $^{90}$Y-labeled antibodies. Additionally, internalization of labeled CD23 antagonists is not required for cell killing, and the local emission of ionizing radiation should be lethal for adjacent tumor cells lacking the target antigen.

Effective single treatment dosages (*i.e.*, therapeutically effective amounts) of $^{90}$Y-labeled CD23 antagonists range from between about 5 and about 75 mCi, more preferably between about 10 and about 40 mCi. Effective single treatment non-marrow ablative dosages of $^{131}$I-labeled antibodies range from between about 5 and about 70 mCi, more preferably between about 5 and about 40 mCi. Effective single treatment ablative dosages (*i.e.*, may require autologous bone marrow transplantation) of $^{131}$I-labeled antibodies range from between about 30 and about 600 mCi, more preferably between about 50 and less than about 500 mCi. In conjunction with a chimeric antibody, owing to the longer circulating half life vis-á-vis murine antibodies, an effective single treatment non-marrow ablative dosages of iodine-131 labeled chimeric antibodies range from between about 5 and about 40 mCi, more preferably less than about 30 mCi. Imaging criteria for. *e.g.*, the $^{111}$In label, are typically less than about 5 mCi.

While a great deal of clinical experience has been gained with $^{131}$I and $^{90}$Y, other radiolabels are known in the art and have been used for similar purposes. Still other radioisotopes are used for imaging. For example, additional radioisotopes which are compatible with the scope of the instant invention include, but are not limited to, $^{123}$I, $^{125}$I, $^{32}$P, $^{57}$Co, $^{64}$Cu, $^{67}$Cu, $^{77}$Br, $^{81}$Rb, $^{81}$Kr, $^{97}$Sr, $^{113}$In, $^{127}$Cs, $^{129}$Cs, $^{132}$I, $^{197}$Hg, $^{203}$Pb, $^{206}$Bi, $^{177}$Lu, $^{186}$Re, $^{212}$Pb, $^{212}$Bi, $^{47}$Sc, $^{105}$Rh, $^{109}$Pd, $^{153}$Sm, $^{188}$Re. $^{199}$Au, $^{225}$Ac, $^{211}$At, and $^{213}$Bi. In this respect alpha, gamma and beta emitters are all compatible with in the instant invention. Further, in view of the instant disclosure it is submitted that one skilled in the art could readily determine which radionuclides are compatible with a selected course of treatment without undue experimentation. To this end, additional radionuclides which have already been used in clinical diagnosis include $^{125}$I, $^{123}$I, $^{99}$Tc, $^{43}$K, $^{52}$Fe, $^{67}$Ga, $^{68}$Ga, as well as $^{111}$In. Antibodies have also been labeled with a variety of radionuclides for potential use in targeted immunotherapy Peirersz et al. Immunol. Cell Biol. 65: 111-125 (1987). These radionuclides include $^{188}$Re and $^{186}$Re as well as $^{199}$Au and $^{67}$Cu to a lesser extent. U.S. Patent No. 5,460,785 provides additional data regarding such radioisotopes and is incorporated herein by reference.

In addition to radionuclides, the CD23 antagonists of the present invention may be conjugated to, or associated with, any one of a number of biological response modifiers, pharmaceutical agents, toxins or immunologically active ligands. Those skilled in the art will appreciate that these non-radioactive conjugates may be assembled using a variety of techniques depending on the selected cytotoxin. For example, conjugates with biotin are prepared e.g. by reacting the CD23 antagonists (i.e. antibodies) with an activated ester of biotin such as the biotin N-hydroxysuccinimide ester. Similarly, conjugates with a fluorescent marker may be prepared in the presence of a coupling agent, e.g. those listed above, or by reaction with an isothiocyanate, preferably fluorescein-isothiocyanate. Conjugates of chimeric antibodies (i.e. IDEC-152) of the invention with cytostatic/cytotoxic substance and metal chelates are prepared in an analogous manner.

As previously alluded to, compatible cytotoxins may comprise a prodrug. As used herein, the term "prodrug" refers to a precursor or derivative form of a pharmaceutically active substance that is less cytotoxic to tumor cells compared to the parent drug and is capable of being enzymatically activated or converted into the more active parent form. Prodrugs compatible with the invention include, but are not limited to, phosphate-containing prodrugs, thiophosphate-containing prodrugs, sulfate containing prodrugs, peptide containing prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs that can be converted to the more active cytotoxic free drug. Further examples of cytotoxic drugs that can be derivatized into a prodrug form for use in the present invention comprise those chemotherapeutic agents described above.

Whether or not the disclosed CD23 antagonists are used in a conjugated or unconjugated form, it will be appreciated that a major advantage of the present invention is the ability to use these antibodies in myelosuppressed patients, especially those who are undergoing, or have undergone, adjunct therapies such as radiotherapy or chemotherapy. In this regard, the unique delivery profile of the CD23 antagonists make them very effective for the administration of radiolabeled conjugates to myelosuppressed cancer patients. As such, the CD23 antagonists are useful in a conjugated or unconjugated form in patients that have previously undergone adjunct therapies such as external beam radiation or chemotherapy. In other preferred embodiments, the CD23 antagonists (again in a conjugated or unconjugated form) may be used in a combined therapeutic regimen with chemotherapeutic agents. Those skilled in the art will appreciate that such therapeutic regimens may comprise the sequential, simultaneous, concurrent or coextensive administration of the disclosed CD23 antagonists and one or more chemotherapeutic agents.

While the CD23 antagonists may be administered as described immediately above, it must be emphasized that in other embodiments conjugated and unconjugated CD23 antagonists may be administered to otherwise healthy cancer patients as a first line therapeutic agent. In such embodiments the CD23 antagonists may be administered to patients having normal or average red marrow reserves and/or to patients that have not, and are not, undergoing adjunct therapies such as external beam radiation or chemotherapy.

However, as discussed above, selected embodiments of the invention comprise the administration of CD23 antagonists to myelosuppressed patients or in combination or conjunction with one or more adjunct therapies such as radiotherapy or chemotherapy (i.e. a combined therapeutic regimen). As used herein, the administration of CD23 antagonists in conjunction or combination with an adjunct therapy means the sequential, simultaneous, coextensive, concurrent, concomitant or contemporaneous administration or application of the therapy and the disclosed antibodies. Those skilled in the art will appreciate that the administration or application of the various components of the combined therapeutic regimen may be timed to enhance the overall effectiveness of the treatment. For example, chemotherapeutic agents could be administered in standard, well known courses of treatment followed within a few weeks by the CD23 antagonists of the present invention. Conversely, cytotoxin associated CD23 antagonists could be administered intravenously followed by tumor localized external beam radiation. In yet other embodiments, the antagonists may be administered concurrently with one or more selected chemotherapeutic agents in a single office visit. A skilled artisan (e.g. an experienced oncologist) would be readily be able to discern effective combined therapeutic regimens without undue experimentation based on the selected adjunct therapy and the teachings of the instant specification.

In this regard it will be appreciated that the combination of the CD23 antagonists (with or without cytotoxin) and a chemotherapeutic agent may be administered in any order and within any time frame that provides a therapeutic benefit to the patient. That is, the chemotherapeutic agent and CD23 antagonist may be administered in any order or concurrently. In selected embodiments the CD23 antagonists of the present invention will be administered to patients that have previously undergone chemotherapy. In yet other embodiments, the CD23 antagonists and the chemotherapeutic treatment will be administered substantially simultaneously or concurrently. For example, the patient may be given the CD23 antagonists while undergoing a course of chemotherapy. In preferred embodiments the CD23 antagonists will be administered within 1 year of any chemotherapeutic agent or treatment. In other preferred embodiments the CD23 antagonists will be administered within 10, 8, 6, 4, or 2 months of any chemotherapeutic agent or treatment. In still other preferred embodiments the CD23 antagonists will be administered within 4, 3, 2 or 1 week of any chemotherapeutic agent or treatment. In yet other embodiments the CD23 antagonists will be administered within 5, 4, 3, 2 or 1 days of the selected chemotherapeutic agent or treatment. It will further be appreciated that the two agents or treatments may be administered to the patient within a matter of hours or minutes (i.e. substantially simultaneously).

Moreover, in accordance with the present invention a myelosuppressed patient shall be held to mean any patient exhibiting lowered blood counts. Those skilled in the art will appreciate that there are several blood count parameters conventionally used as clinical indicators of myelosuppresion and one can easily measure the extent to which myelosuppresion is occurring in a patient. Examples of art accepted myelosuppression measurements are the Absolute Neutrophil Count (ANC) or platelet count. Such myelosuppression or partial myeloablation may be a result of various biochemical disorders or diseases or, more likely, as the result of prior chemotherapy or radiotherapy. In this respect, those skilled in the art will appreciate that patients who have undergone traditional chemotherapy typically exhibit reduced red marrow reserves.

More specifically conjugated or unconjugated CD23 antagonists of the present invention may be used to effectively treat patients having ANCs lower than about 2000/mm$^3$ or platelet counts lower than about 150,000/ mm$^3$. More preferably the CD23 antagonists of the present invention may be used to treat patients having ANCs of less than about 1500/ mm$^3$, less than about 1000/mm$^3$ or even more preferably less than about 500/ mm$^3$. Similarly, the CD23 antagonists of the present invention may be used to treat patients having a platelet count of less than about 75,000/mm$^3$, less than about 50,000/mm$^3$ or even less than about 10,000/mm$^3$. In a more general sense, those skilled in the art will easily be able to determine when a patient is myelosuppressed using government implemented guidelines and procedures.

As indicated above, many myelosuppressed patients have undergone courses of treatment including chemotherapy, implant radiotherapy or external beam radiotherapy. In the case of the latter, an external radiation source is for local irradiation of a malignancy. For radiotherapy implantation methods, radioactive reagents are surgically located within the malignancy, thereby selectively irradiating the site of the disease. In any event, the disclosed antagonists may be used to treat neoplastic disorders in patients exhibiting myelosuppression regardless of the cause.

It will further be appreciated that the CD23 antagonists of the instant invention may be used in conjunction or combination with any chemotherapeutic agent or agents (e.g. to provide a combined therapeutic regimen) that eliminates, reduces, inhibits or controls the growth of neoplastic cells *in vivo*. As used herein the terms "chemotherapeutic agent" or "chemotherapeutics "shall be held to mean any therapeutic compound that is administered to treat or prevent the growth of neoplastic cells *in vivo*. In particular, chemotherapeutic agents compatible with the present invention comprise both "traditional" chemotherapeutic agents such as small molecules and more recently developed biologics such as antibodies, cytokines, antisense molecules, etc. that are used to reduce or retard the growth of malignant cells. Particularly preferred chemotherapeutic agents that are compatible for use with the disclosed CD23 antagonists include commercially available antibodies directed to tumor associated antigens such as Rituxan®, Zevalin™, Herceptin®, Lymphocide®, Campath®, etc. In additional preferred embodiments, antineoplastic antibodies undergoing clinical trials may be used in combination with CD23 antagonists. For example, IDEC-114 and IDEC-131 (IDEC Pharmaceuticals, San Diego CA) directed to the B7 antigen and CD40L antigen respectively, may be used with the disclosed antagonists to treat selected neoplasms. In this regard Lym-1 (Peregrin Pharmaceuticals, Tustin CA) and Erbitux™ (Imclone Pharmaceuticals, Cambridge MA) are also compatible with the instant invention. Other biologic chemotherapeutic agents that are compatible include cytokines such as lymphokines, interleukins, tumor necrosis factors and growth factors. The CD23 antagonists may also be used in conjunction with immunosuppressive agents, prodrugs or cytotoxic agents for the treatment of selected malignancies.

Chemotherapeutic antibodies that are particularly useful in combination with CD23 antagonists include Y2B8 and C2B8 (Zevalin™ & Rituxan®) IDEC-114 and IDEC-131, (IDEC Pharmaceutical Corp., San Diego), Lym 1 and Lym 2, LL2 (Immunomedics Corp., New Jersey), HER2 (Herceptin®, Genentech Inc., South San Francisco), B1 (Bexxar®, Coulter Pharm., San Francisco), MB1, BH3, B4, B72.3 (Cytogen Corp.), CC49 (National Cancer Institute) and 5E10 (University of Iowa). Rituxan is the first FDA-approved monoclonal antibody for treatment of human B-cell lymphoma (see U.S. Patent Nos. 5,843,439; 5,776,456 and 5,736,137 each of which is incorporated herein by reference). Y2B8 is the murine parent of C2B8. Rituxan is a chimeric, anti-CD20 monoclonal antibody (MAb) which is growth inhibitory and reportedly sensitizes certain lymphoma cell lines for apoptosis by chemotherapeutic agents *in vitro.* The antibody efficiently binds human complement, has strong FcR binding, and can effectively kill human lymphocytes *in vitro* via both complement dependent (CDC) and antibody-dependent (ADCC) mechanisms (Reff et al., Blood 83: 435-445 (1994)). Those skilled in the art will appreciate that any antibody directed to common tumor associated or immunomodulatory antigens such as CD20, CD22, B7 or CD40L is compatible with the instant invention and may be used in combination with the disclosed antagonists.

More "traditional" chemotherapeutic agents useful in the instant invention include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime nitrogen mustards such as chiorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenished such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2′,2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, NJ) and doxetaxel (Taxotere, Rhone-Poulenc Rorer, Antony, France); chlorambucil, gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, troxifene, keoxifene, LY117018, onapristone, and toremifene (Fareston); and antiandrogens such as flutamide, nilutamide, bicalutamide, leuprolide, and goserelin; and pharmaceutically acceptable salts, acids or derivatives of any of the above.

Compatible chemotherapeutic regimens of comprise combinations of drugs. The four-drug combination MOPP (mechlethamine (nitrogen mustard), vincristine (Oncovin), procarbazine and prednisone) is very effective in treating various types of lymphoma and comprises a preferred embodiment of the present invention. In MOPP-resistant patients, ABVD (*e.g.*, adriamycin, bleomycin, vinblastine and dacarbazine), ChlVPP (chlorambucil, vinblastine, procarbazine and prednisone), CABS (lomustine, doxorubicin, bleomycin and streptozotocin), MOPP plus ABVD, MOPP plus ABV (doxorubicin, bleomycin and vinblastine) or BCVPP (carmustine, cyclophosphamide, vinblastine, procarbazine and prednisone) combinations can be used. Arnold S. Freedman and Lee M. Nadler, Malignant Lymphomas, in HARRISON'S PRINCIPLES OF INTERNAL MEDICINE 1774-1788 (Kurt J. Isselbacher et al., eds., 13th ed. 1994) and V. T. DeVita *et al.,* (1997) and the references cited therein for standard dosing and scheduling. These therapies can be used unchanged, or altered as needed for a particular patient, in combination with the CD23 antagonists as described herein.

Additional regimens that are useful in the context of the present invention include use of single alkylating agents such as cyclophosphamide or chlorambucil, or combinations such as CVP (cyclophosphamide, vincristine and prednisone), CHOP (CVP and doxorubicin), C-MOPP (cyclophosphamide, vincristine, prednisone and procarbazine), CAP-BOP (CHOP plus procarbazine and bleomycin), m-BACOD (CHOP plus methotrexate, bleomycin and leucovorin), ProMACE-MOPP (prednisone, methotrexate, doxorubicin, cyclophosphamide, etoposide and leucovorin plus standard MOPP), ProMACE-CytaBOM (prednisone, doxorubicin, cyclophosphamide, etoposide, cytarabine, bleomycin, vincristine, methotrexate and leucovorin) and MACOP-B (methotrexate, doxorubicin, cyclophosphamide, vincristine, fixed dose prednisone, bleomycin and leucovorin). Those skilled in the art will readily be able to determine standard dosages and scheduling for each of these regimens. CHOP has also been combined with bleomycin, methotrexate, procarbazine, nitrogen mustard, cytosine arabinoside and etoposide. Other compatible chemotherapeutic agents include, but are not

limited to, 2-chlorodeoxyadenosine (2-CDA), 2'-deoxycoformycin and fludarabine.

For patients with intermediate- and high-grade NHL, who fail to achieve remission or relapse, salvage therapy is used. Salvage therapies employ drugs such as cytosine arabinoside, cisplatin, etoposide and ifosfamide given alone or in combination. In relapsed or aggressive forms of certain neoplastic disorders the following protocols are often used: IMVP-16 (ifosfamide, methotrexate and etoposide), MIME (methyl-gag, ifosfamide, methotrexate and etoposide), DHAP (dexamethasone, high dose cytarabine and cisplatin), ESHAP (etoposide, methylpredisolone, HD cytarabine, cisplatin), CEPP(B) (cyclophosphamide, etoposide, procarbazine, prednisone and bleomycin) and CAMP (lomustine, mitoxantrone, cytarabine and prednisone) each with well known dosing rates and schedules. The amount of chemotherapeutic agent to be used in combination with the CD23 antagonists of the instant invention may vary by subject or may be administered according to what is known in the art. See for example, Bruce A Chabner et al., Antineoplastic Agents, in GOODMAN & GILMAN'S THE PHARMACOLOGICAL BASIS OF THERAPEUTICS 1233-1287 ((Joel G. Hardman et al., eds., 9th ed. 1996).

The term "immunosuppressive agent" as used herein for adjunct therapy refers to substances that act to suppress or mask the immune system of the mammal being treated herein. This would include substances that suppress cytokine production, downregute or suppress self-antigen expression, or mask the MHC antigens. Example of such agents include 2-amino-6-aryl-5-substituted pyrimidines (see U.S. Pat. No. 4,665,077, the disclosure of which is incorporated herein by reference), azathioprine; cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e.g.*, prednisone, methylprednisolone, and dexamethasone; cytokine or cytokine receptor antagonists including anti-interferon antibodies, anti-tumor necrosis factor-$\alpha$ antibodies, anti-tumor necrosis factor-$\beta$ antibodies, anti-interleukin-2 antibodies and anti-IL-2 receptor antibodies; anti-LFA-1 antibodies, including anti-CD1 la and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90), streptolanase; TGF-$\beta$; streptodornase; RNA or DNA from the host; FK506; RS-61443; deoxyspergualin; rapamycin; T-cell receptor (Cohen et al., U.S. Pat. No. 5,114,721); T-cell receptor fragments (Offner et al., Science, 251: 430-432 (1991); WO 90/11294; laneway, Nature, 341: 482 (1989); and WO 91/01133); and T cell receptor antibodies (EP 340,109) such as T10B9.

The term "cytotoxic agent" as used herein refers to a substance that inhibits or prevents the function of cells and/or causes destruction of cells. The term is intended to include radioactive isotopes, chemotherapeutic agents, and toxins such as small molecule toxins or enzymatically active toxins of bacterial, fungal, plant or animal origin, or fragments thereof.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factors; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-13; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocytemacrophage-CSF (GM-CSF); and granulocyte-CSF (G-CSF); interleukins (ILs) such as IL-1, IL-1a, IL-2, IL-g, IL-4, IL-5, IL-6, IL-7, IL-8, IL-9, IG-11, IL-12, IL-15; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokine.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g.*, Wilman, "prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, 13-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the antagonists disclosed herein and, optionally, a chemotherapeutic agent) to a mammal. The components of the liposome are commonly arranged in a bilayer formation, similar to the lipid arrangement of biological membranes.

As previously discussed, the antagonists of the present invention, immunoreactive fragments or recombinants thereof may be administered in a pharmaceutically effective amount for the *in vivo* treatment of mammalian malignancies. In this regard, it will be appreciated that the disclosed antagonists will be formulated so as to facilitate administration and promote stability of the active agent. Preferably, pharmaceutical compositions in accordance with the present invention comprise a pharmaceutically acceptable, non-toxic, sterile carrier such as physiological saline, non-toxic buffers, preservatives and the like. For the purposes of the instant application, a pharmaceutically effective amount of the CD23 antagonist, immunoreactive fragment or recombinant thereof, shall be held to mean an amount sufficient to achieve effective binding with the CD23 antigen on neoplastic cells and provide for an increase in the death of those cells. Of course, the pharmaceutical compositions of the present invention may be administered in single or multiple doses to provide for a pharmaceutically effective amount of the CD23 antagonist.

More specifically, they the disclosed antagonists and methods should be useful for reducing tumor size, inhibiting tumor growth and/or prolonging the survival time of tumor-bearing animals. Accordingly, this invention also relates to a method of treating tumors in a human or other animal by administering to such human or animal an effective, non-toxic amount of the CD23 antagonist. One skilled in the art would be able, by routine experimentation, to determine what an effective, non-toxic amount of antagonist would be for the purpose of treating malignancies. For example, a therapeutically active amount of antagonist may vary according to factors such as the disease stage (*e.g.*, stage I versus stage IV), age, sex, medical complications (*e.g.*, immunosuppressed conditions or diseases) and weight of the subject, and the ability of the antagonist to elicit a desired response in the subject. The dosage regimen may be adjusted to provide the optimum therapeutic response. For example, several divided doses may be administered daily, or the dose may be proportionally reduced as indicated by the exigencies of the therapeutic situation. Generally, however, an effective dosage is expected to be in the range of about 0.05 to 100 milligrams per kilogram body weight per day and more preferably from about 0.5 to 10, milligrams per kilogram body weight per day.

In keeping with the scope of the present disclosure, the antagonists of the invention may be administered to a human or other animal in accordance with the aforementioned methods of treatment in an amount sufficient to produce such effect to a therapeutic or prophylactic degree. The antagonists of the invention can be administered to such human or other animal in a conventional dosage form prepared by combining the antagonist of the invention with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables. Those skilled in the art will further appreciate that a cocktail comprising one or more species of antagonists according to the present invention may prove to be particularly effective.

Methods of preparing and administering the CD23 antagonist are well known to or readily determined by those skilled in the art. The route of administration of the antagonist of the invention may be oral, parenteral, by inhalation or topical. The term parenteral as used herein includes intravenous, intraarterial, intraperitoneal, intramuscular, subcutaneous, rectal or vaginal administration. The intravenous, intraarterial, subcutaneous and intramuscular forms of parenteral administration are generally preferred. While all these forms of administration are clearly contemplated as being within the scope of the invention, a preferred administration form would be a solution for injection, in particular for intravenous or intraarterial injection or drip. Usually, a suitable pharmaceutical composition for injection may comprise a buffer (e.g. acetate, phosphate or citrate buffer), a surfactant (e.g. polysorbate), optionally a stabilizer agent (e.g. human albumine), etc. However, in other methods compatible with the teachings herein, the antagonists can be delivered directly to the site of the malignancy site thereby increasing the exposure of the neoplastic tissue to the therapeutic agent.

Preparations for parenteral administration includes sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. In the subject invention, pharmaceutically acceptable carriers include, but are not limited to, 0.01-0.1M and preferably 0.05M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers. electrolyte replenishers, such as those based on Ringer's dextrose, and the like. Preservatives and other additives may also be present such as for example, antimicrobials, antioxidants, chelating agents, and inert gases and the like.

Those of skill in the art will appreciate that pharmaceutical compositions suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, for example, water, ethanol, polyol (*e.g.*, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

More particularly, therapeutic formulations comprising antagonists used in accordance with the present invention are prepared for storage by mixing an antagonist having the desired degree of purity with optional pharmaceutically acceptable carriers, excipients or stabilizers *(*Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes *(e.g.* Zn-protein complexes); and/or non-ionic surfactants such as TWEENTM, PLURONICSTM or polyethylene glycol (PEG).

Lyophilized formulations adapted for subcutaneous administration are described in W097/04801. Such lyophilized formulations may be reconstituted with a suitable diluent to a high protein concentration and the reconstituted formulation may be administered subcutaneously to the mammal to be treated herein.

The formulation herein may also contain more than one active compound as necessary for the particular indication being treated, preferably those with complementary activities that do not adversely affect each other. For example, it may be desirable to further provide a chemotherapeutic agent, cytokine or immunosuppressive agent *(e.g.* one which acts on T cells, such as cyclosporin or an antibody that binds T cells, *e.g.* one which binds LFA-1). The effective amount of such other agents depends on the amount of antagonist present in the formulation, the type of disease or disorder or treatment, and other factors discussed above. These are generally used in the same dosages and with administration routes as used hereinbefore or about from 1 to 99% of the heretofore employed dosages.

The active ingredients may also be entrapped in microcapsules prepared, for example, by 30 coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington 's Pharmaceutical Sciences 16th edition, Osol, A. Ed. (1980).

Sustained-release preparations may also be prepared. Suitable examples of sustained release preparations include semipermeable matrices of solid hydrophobic polymers containing the antagonist, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymer of L-glutamic acid and γ ethyl-L-glutamate, noir degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. The formulations to be used for in vivo administration must be sterile. This is readily accomplished by filtration through sterile filtration membranes.

Prevention of the action of microorganisms can further be achieved by various antibacterial and antifungal agents, for example, parabens, chlorobutanol, phenol, ascorbic acid, thimerosal and the like. In many cases, it will be preferable to include isotonic agents, for example, sugars, polyalcohols, such as mannitol, sorbitol, or sodium chloride in the composition. Prolonged absorption of the injectable compositions can be brought about by including in the composition an agent which delays absorption, for example, aluminum monostearate and gelatin.

In any case, sterile injectable solutions can be prepared by incorporating an active compound (*e.g.*, an antagonist by itself or in combination with other active agents) in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yields a powder of an active ingredient plus any additional desired ingredient from a previously sterile-filtered solution thereof.

The preparations for injections are processed and filled into containers such as ampoules, bags, bottles, syringes or vials, and sealed under aseptic conditions according to methods known in the art. The containers may be formed from a variety of materials such as glass or plastic and holds, contains or has dispersed therein a composition which is effective for treating the disease or disorder of choice. In addition, the container may have a sterile access port (for example the container may be an intravenous solution bag or a vial having a stopper pierceable by a hypodermic injection needle). These preparations may be packaged and sold in the form of a kit such as those described in co-pending U.S.S.N. 09/259,337 and U.S.S.N. 09/259,338 each of which is incorporated herein by reference. Such articles of manufacture will preferably have labels or package inserts indicating that the associated compositions are useful for treating a subject suffering from, or predisposed to, cancer, malignancy or neoplastic disorders (e.g. chronic lymphocytic leukemia). The term "package insert" is used to refer to instructions customarily included in commercial packages of therapeutic products, that contain information about the indications, usage, dosage, administration, contraindications and/or warnings concerning the use of such therapeutic products. The article of manufacture may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

As discussed in detail above, the present invention provides compounds, compositions, kits and methods for the treatment of neoplastic disorders in a mammalian subject in need of treatment thereof. Preferably, the subject is a human. While the instant invention is particularly effective in the treatment CD23+ hematalogic malignancies including chronic lymphocytic leukemia, the disclosed antagonists and methods may be used to treat any CD23+ neoplasms. In this respect the CD23+ neoplastic disorder (e.g., cancers and malignancies) may comprise solid tumors such as melanomas, gliomas, sarcomas, and carcinomas as well as myeloid or hematologic malignancies such as lymphomas and leukemias. The disclosed invention may be used to prophylactically or therapeutically treat any neoplasm comprising CD23 antigenic marker that allows for the targeting of the cancerous cells by the antagonist. Exemplary cancers that may be treated include, but are not limited to, prostate, colon, skin, breast, ovarian, lung and pancreatic. In preferred embodiments the antagonist may be used to treat More particularly, the antibodies of the instant invention may be used to treat Kaposi's sarcoma, CNS neoplasms (capillary hemangioblastomas, meningiomas and cerebral metastases), mastocyloma, melanoma, gastrointestinal and renal sarcomas, rhabdomyosarcoma, glioblastoma (preferably glioblastoma multiforme), leiomyosarcoma, retinoblastoma, papillary cystadenocarcinoma of the ovary, Wilm's tumour or small cell lung carcinoma. It will be appreciated that appropriate antagonists may be derived for CD23 as expressed on each of the forgoing neoplasms without undue experimentation in view of the instant disclosure.

For purposes of clarification "Mammal" refers to any animal classified as a mammal, including humans, domestic and farm animals, and zoo, sports, or pet animals, such as dogs, horses, cats, cows, *etc.* Preferably, the mammal is human.

"Treatment" refers to both therapeutic treatment and prophylactic or preventative measures. Those in need of treatment include those already with the disease or disorder as well as those in which the disease or disorder is to be prevented. Hence, the mammal may have been diagnosed as having the disease or disorder or may be predisposed or susceptible to the disease.

As previously discussed the methods, compositions and articles of manufacture of the present invention are particularly useful in the treatment of chronic lymphocytic leukemia. However, the treatment of other CD23+ hematologic malignancies may also be effected using the disclosed methods and are clearly within the scope of the instant invention. In this respect, exemplary hematologic malignancies that are amenable to treatment with the disclosed invention include small T cell lymphomas, lymphocytic lymphoma, mantle cell lymphoma, Hodgkins and non-Hodgkins lymphoma as well as leukemias, including ALL-L3 (Burkitt's type leukemia), acute T cell leukemia, chronic myelogenous leukemia and monocytic cell leukemias.

It will be further be appreciated that the compounds and methods of the present invention are particularly effective in treating a variety of B-cell lymphomas, including low grade/ NHL follicular cell lymphoma (FCC), mantle cell lymphoma (MCL), diffuse large cell lymphoma (DLCL), small lymphocytic (SL) NHL, intermediate grade/ follicular NHL, intermediate grade diffuse NHL, high grade immunoblastic NHL, high grade lymphoblastic NHL, high grade small non-cleaved cell NHL, bulky disease NHL, Waldenstrom's Macroglobulinemia, lymhoplasmacytoid lymphoma (LPL), mantle cell lymphoma (MCL), follicular, lymphoma (FL), diffuse large cell lymphoma (DLCL), Burkitt's lymphoma (BL), AIDS-related lymphoma, monocytic B cell lymphoma, angioimmunoblastic lymphoadenopathy, small lymphocytic, follicular, diffuse large cell, diffuse small cleaved cell, large cell immunoblastic lymphoblastoma, small, non-cleaved, Burkitt's and non-Burkitt's, follicular, predominantly large cell; follicular, predominantly small cleaved cell; and follicular, mixed small cleaved and large cell lymphomas. See, Gaidono et al., "Lymphomas", IN CANCER: PRINCIPLES & PRACTICE OF ONCOLOGY, Vol. 2: 2131-2145 (DeVita et al., eds., 5th ed. 1997). It should be clear to those of skill in the art that these lymphomas will often have different names due to changing systems of classification, and that patients having lymphomas classified under different names may also benefit from the combined therapeutic regimens of the present invention. In addition to the aforementioned neoplastic disorders, it will be appreciated that the disclosed invention may advantageously be used to treat additional malignancies expressing the CD23 antigen.

The foregoing description will be more fully understood with reference to the following examples. Such examples, are, however, demonstrative of preferred methods of practicing the present invention and are not limiting of the scope of the invention or the claims appended hereto.

Several antibodies were used to conduct the experiments set forth in the Examples below. As previously indicated, IDEC-152 (p5E8) is a Primatized® anti-human CD23 MAb that contains human gamma 1. heavy chain (Lot # ZC152-02) and Rituxan® (rituximab) is an anti-human CD20 specific mouse-human gamma 1 chimeric antibody (Lot E9107A1; Lot D9097A1). Other antibodies used include the murine anti-human CD23 MAb labeled with PE (Cat # 33615X, BD Pharmingen, San Diego, CA) and the primatized anti-human CD4 MAb CE9.1, with human gamma 1 chain (Lot M2CD4156). RF-2 a fully human antibody specific to RSV fusion protein was used as an isotype (IgG1) matched antibody control.

## Example 1

### Expression CD23 in B-lymphoma and B-CLL cells

The expression of CD23 in several lymphoma cell lines was determined by flowcytometry. More particularly, CD23 expression was evaluated by flowcytometry using anti-CD23 PE-labeled antibody (BD Biosciences, Cat.No; 33615X). The relative fluorescence intensity (RFI) of antibody binding was determined by comparing the mean fluorescence intensity of anti-CD23-PE antibody binding to cells to that of the mean fluorescence intensity of the PE-labeled calibration beads (QuantiBrite). The relative expression of CD23 was calculated as RF1 (sample) ÷ RFI of the SKW cells.

CD20- and B7-expressing B-lymphoma cell lines (SKW, SB, Daudi, Raji, Ramos and DHL-4 cells) were cultured in complete medium. Complete medium is RPMI 1640 medium (Irvine Scientific, Santa Ana, CA) supplemented with 10% heat inactivated FBS (Hyclone), 2 mM 1-glutamine, 100 units/ml of penicillin, and 100 ug/ml of streptomycin. The SKW cell line is Epstein-Barr virus (EBV) positive and can be induced to secrete IgM (SKW 6.4, ATCC). The SB cell line originated from a patient with acute lymphoblastic leukemia and is positive for EBV (CCL-120, ATCC). The Daudi cell line was isolated from a patient with Burkitt's lymphoma (CCL-213, ATCC). The Raji and Ramos cell lines was also isolated from Burkitt's lymphoma patients (CCL-86, CRL-1596, ATCC). The DHL-4 was isolated from a patient diagnosed with diffuse histiocytic lymphoma (Epstein et al., Cancer. 1978, 42:2379).

Of those tested, 3 out 6 cell lines exhibited CD23 expression. As shown immediately below in Table 1, CD23 expression in SKW and SB cells was roughly comparable, whereas Raji cells showed only a marginal level of antigen expression equivalent to 10% of the CD23 levels expressed in SKW cells.

In addition to determining the levels of CD23 expression, the same cell lines were assayed to establish their level of susceptibility to apoptosis induced by anti-CD23 antibodies. Specifically, the induction of apoptosis in each of the six cell lines was determined using a caspase-3 assay.

Those skilled in the art will appreciate that the caspase-3 assay measures the activation of caspase-3 enzyme, a critical early event of apoptosis induced death. In the instant example, SKW cells at $0.5 \times 10^6$ cells/ml density in culture media (RPMI-2% FBS) were incubated with 10 ug/mL of IDEC-152 at 4°C in cell culture tubes on ice. After 1 hour of incubation the unbound antibody in the media was removed by centrifugation. The cells were resuspended in growth media in appropriate volumes and added into 24 well tissue culture plates ($1.5 \times 10^6$ cells/well) with and without the addition of goat anti-human Ig-Fcγ specific secondary antibody (15μg/ml) as a crosslinker. Following incubation for 18 hours, cells were harvested and analyzed for apoptosis by flowcytometry. In particular, the cells were washed and fixed at 4°C using Cytofix (Cytofix/Cytoperm™ Kit, Pharmingen Cat # 2075KK). After 20 min of fixation, cells were washed and 15μl of affinity purified PE-conjugated polyclonal rabbit anti-caspase-3 antibody (Pharmingen Cat. # 67345) and 50 μl of Cytoperm were added. Cells were incubated on ice in the dark for 30 min. After incubation cells were washed once and resuspended in Cytoperm wash. Flow cytometry data was acquired on FACScan and analyzed using WinList software from Verity Software House. The results are presented in Table 1 immediately below.

**Table 1**

| Induction of Apoptosis in CD23+ B lymphoma Cell Lines | | | |
|---|---|---|---|
| Cell Line | Origin | Relative Expression of CD23 | Apoptosis |
| SKW | Burkitt's lymphoma | 100 | 61 |
| SB | Acute Lymphoblastic Leukemia | 110 | 42.1 |
| DHL-4 | Diffuse Histiocytic Lymphoma | 0 | 0 |
| Daudi | Burkitt's Lymphoma | 0 | 0 |
| Raji | Burkitt's Lymphoma | 10 | 0 |
| Ramos | Burkitt's Lymphoma | 0 | 0 |

The results set forth above show that those cell lines expressing high levels of CD23 will undergo programmed cell death upon exposure to cross-linked antibodies to CD23. Conversely, cells that do not express CD23 at high levels do not undergo extensive apoptosis. Accordingly, the instant example provides for the identification of selected cell lines that may serve as clinically relevant models for CD23+ B cell malignancies (e.g., SKW cells and SB cells).

**Example 2**

**Expression CD23 in CLL cells**

In order to demonstrate the clinical applicability of the present invention, the expression of CD23 on several different CLL samples (31 patients) was tested in whole blood by flowcytometry. Using appropriate reagents, flowcytometry was performed as substantially described in Example 1. In this respect, the expression of CD20 and CD23 was determined on gated cells that were CD19+ positive. Specifically, PE labeled anti-CD20 (BD Biosciences /Pharmingen, Cat # 555623) and anti-CD23 (BD Biosciences /Pharmingen, Cat # 33615X) monoclonal antibodies were used to detect CD20 and CD23 molecules respectively.

In all patients, the expression of both CD20 and CD23 antigen was detected in CD19+B cells as shown immediately below in Table 2. Patients expressing high CD20 levels expressed varying degrees of CD23 antigen in their CLL samples. The levels were determined by the percentage CD19+ cells and mean fluorescence intensity (MFI). However, even in patients expressing low levels of CD20 the measured values show that relatively high levels of CD23 may be expressed. These findings indicate that the CD23 antigen may prove to be an extremely attractive target for therapeutically relevant antibodies such as those disclosed in the instant invention.

**Table 2**

| Expression of CD23 and CD20 in B-CLL cells from CLL patients. | | | | |
|---|---|---|---|---|
| **Patient Case #** | CD20 Expression | | CD23 Expression | |
| | MFI | **% Positive** | MFI | **%Positive** |
| **CD20 high** | | | | |
| 1 | 92 | 79 | 76 | 51 |
| 2 | 67 | 79 | 45 | 47 |
| 3 | 385 | 82 | 113 | 77 |
| 4 | 241 | 92 | 189 | 87 |
| 5 | 313 | 88 | 89 | 86 |
| 7 | 375 | 89 | 743 | 91 |
| 9 | 255 | 76 | 97 | 48 |
| 11 | 649 | 92 | 73 | 71 |
| 12 | 109 | 96 | 81 | 88 |
| 13 | 311 | 94 | 403 | 95 |
| 14 | 151 | 92 | 76 | 58 |
| 15 | 667 | 71 | 777 | 81 |
| 19 | 148 | 93 | 154 | 88 |
| 21 | 84 | 83 | 45 | 43 |
| 28 | 122 | 69 | 164 | 72 |
| **CD20 low** | | | | |
| 6 | 52 | 35 | 88 | 76 |
| 8 | 129 | 26 | 157 | 32 |
| 10 | 116 | 53 | 327 | 83 |
| 16 | 198 | 25 | 181 | 31 |
| 17 | 125 | 34 | 199 | 24 |
| 18 | 66 | 54 | 96 | 91 |
| 20 | 48 | 63 | 128 | 79 |
| 22 | 138 | 62 | 173 | 54 |
| 23 | 163 | 15 | 356 | 25 |
| 24 | 115 | 37 | 89 | 24 |
| 25 | 17 | 41 | 86 | 49 |

(continued)

| CD20 low | | | | |
|---|---|---|---|---|
| 26 | 302 | 55 | 302 | 58 |
| 27 | 289 | 55 | 195 | 57 |
| 29 | 107 | 26 | 193 | 29 |
| 30 | 109 | 43 | 356 | 57 |
| 31 | 105 | 36 | 292 | 46 |

## Example 3

### Binding of IDEC-152 to CD23+ cells

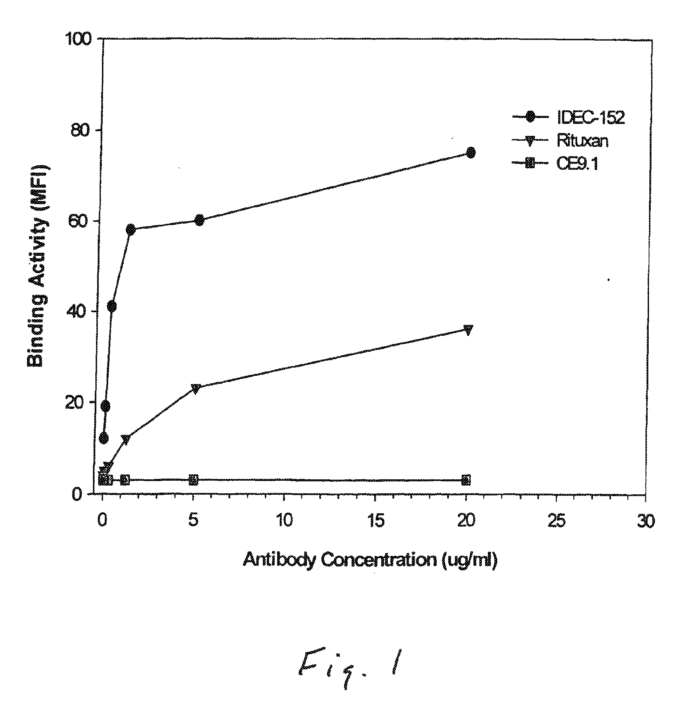

To further demonstrate the advantages of the present invention, the binding activity of IDEC-152 to CD23 on SKW lymphoma cells was determined by flowcytometry as set forth in the previous examples. As indicated above, SKW cells may be used to provide a clinically relevant model for CD23+ malignancies including CLL. The results of the assay are set forth in Figure 1, which shows the specific binding of Rituxan and IDEC-152 to SKW cells in a concentration dependent fashion. The binding activity is measured using mean fluorescence intensity and shows that the SKW cells bind substantially higher levels of anti-CD23 antibodies than anti-CD20 antibodies. This indicates that, in certain cell lines and tumors, CD23 may exhibit a higher epitope density than other markers such as CD20. As expected, isotype-matched control antibody of irrelevant specificity (CE9.1, directed to CD4) did not bind to SKW. This Example, and the corresponding results set forth in Figure 1, confirm the desirability of using CD23 as a target for therapeutic antibodies in the treatment of selected neoplasms.

## Example 4

### IDEC-152 mediates ADCC activity in CD23+ cells

The ability of IDEC-52 to mediate ADCC of tumor cells was determined. In the ADCC assay lymphoma cells (SKW or SB) and activated human peripheral monocytes (PBMC) were used as targets and effector cells, respectively. PBMC were isolated from whole blood of healthy donors using Histopaque (Sigma-Aldrich Corp., St. Louis, MO). The PBMC were cultured at a concentration of $5 \times 10^6$ cells/ml in complete medium with 20 U/ml recombinant human IL-2 (Invitrogen, Carlsbad, CA) in 75 $cm^2$ tissue culture flasks at 37°C and 5% $CO_2$. After overnight culture, $1 \times 10^6$ SKW or SB target cells were labeled with 150 $\mu$Ci of $^{51}$Cr (Amersham Pharmacia Biotech, Piscataway, NJ) for 1 hour at 37°C and 5% $CO_2$. The cells were washed four times and resuspended in 5 ml of complete medium; 50 $\mu$l of cell suspension was dispensed into each well containing equal volume of test or control antibodies.

Rituximab (Lot E9107A1) or IDEC-152 (Lot ZC 152-02) were used as test antibodies. Isotype ($IgG_1$) matched CE9.1 (Lot M2CD4156) antibody of irrelevant specificity was used as the control. All wells were plated in triplicate into a 96 well, round bottom tissue culture plate. The effector cells were harvested, washed once with complete medium, and added at $1 \times 10^6$ cells in 100 $\mu$l volume per well to obtain a 50:1 effector to target ratio. The following control wells were also included in triplicate: target cell incubated with 100 $\mu$l complete medium to determine spontaneous release and target cell incubated with 100 $\mu$l 0.5% Triton X-100 (Sigma-Aldrich Corp.) to determine maximum release. The culture was incubated for 4 hours at 37°C and 5% $CO_2$ and the $^{51}$Cr released in the culture supernatant due to cell lysis was determined by a gamma counter (ISODATA). The cytotoxicity was expressed as the percentage of specific lysis and calculated as follows:

$$1 - \frac{^{51}\text{Cr release of test samples} - \text{spontaneous } ^{51}\text{Cr release}}{\text{Maximum } ^{51}\text{Cr release} - \text{spontaneous } ^{51}\text{Cr release}} \times 100$$

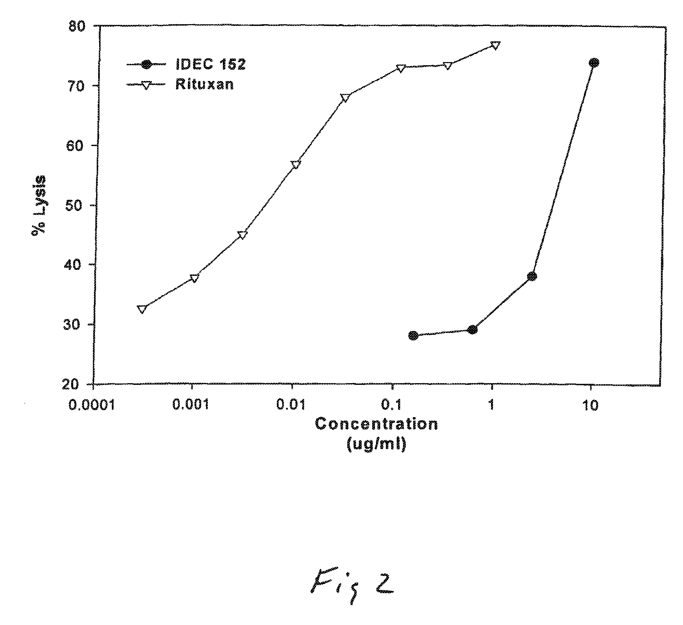

Figure 2 shows the result of this assay and more particularly the ADCC activity of IDEC-152 and Rituxan on CD20+/CD23+ SKW cells. Both IDEC-152 and Rituxan showed a dose-dependent killing of SKW cells with a maximum killing of 75% achieved at 10 $\mu$g/ml and 1 $\mu$g/ml antibody concentrations respectively, indicating that Rituxan is more potent than IDEC-152 in mediating ADCC in this particular cell line. However, the antibody binding activity shown in Figure 1 suggests that the potency differences between IDEC-152 and Rituxan is not entirely related to the antibody binding efficiency or to the epitope density of CD23 and CD20. As expected, only background levels (<10%) of ADCC were observed with isotype matched human CE9.1 control antibody (not shown).

**Example 5**

**IDEC-152 synergizes with Rituxan to mediate ADCC activity**

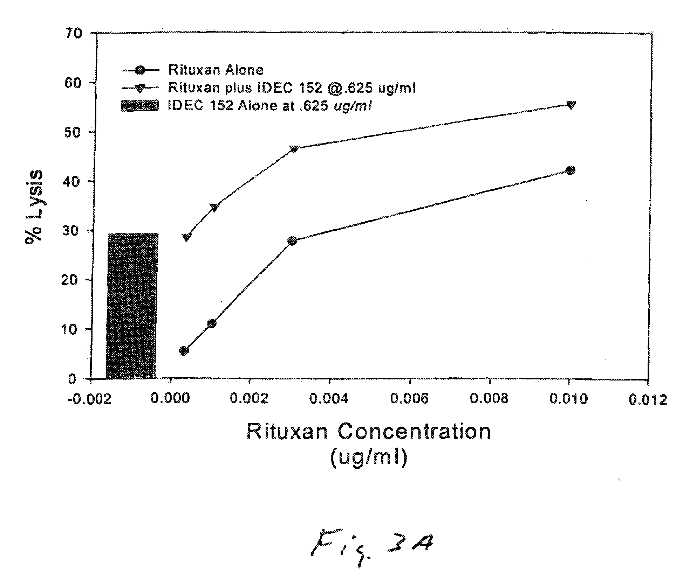

In order to demonstrate the synergistic aspects of the present invention with different antibodies, the combination of IDEC-152 and Rituxan on ADCC mediated *in vitro* tumor killing was investigated. SKW cells were incubated with IDEC-152 at two concentrations (0.625 μg/ml & 2.5 μg/ml) either by itself or in combination with varying concentrations of Rituxan. The same concentrations of Rituxan alone were run as a control. Resulting ADCC activity on the tumor cells was measured substantially as set forth in Example 4 and is shown in Figs. 3A (0.625 μg/ml IDEC-152) & 3B (2.5 μg/ml IDEC-152).

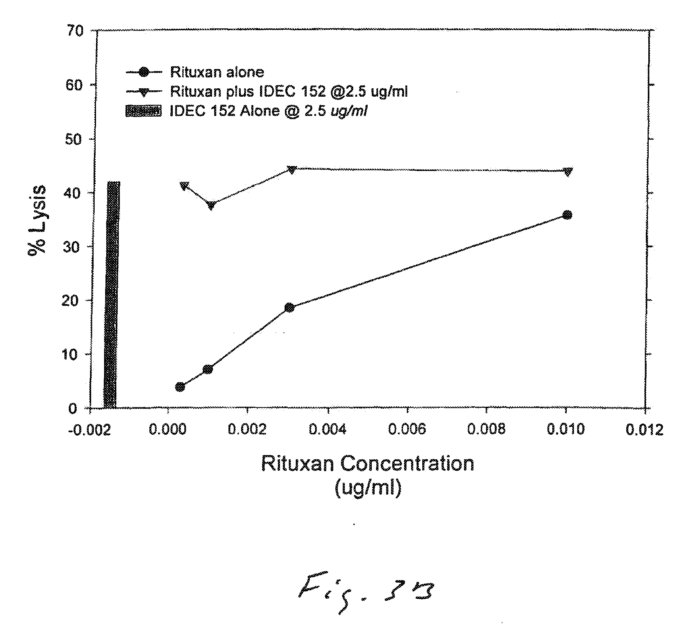

The results of the assays shows that the combination of IDEC-152 with Rituxan increases ADCC activity beyond the activity achieved with either agent individually. More particularly, Fig. 3A shows that the combination of the antibodies results in substantially higher levels of cell lysis at all concentrations of Rituxan than either IDEC-152 or Rituxan alone. Conversely, as shown in Fig. 3B, IDEC-152 is such an efficient mediator of ADCC that at higher concentrations (i.e. 2.5 μg/ml) any potential synergistic effect is swamped by the anti-CD23 antibody. That is, as shown in Fig. 3B, no change in cytotoxicity was observed at high concentrations of either IDEC-152 or Rituxan. This Example graphically illustrates the ability of the present invention to dramatically enhance the effectiveness of proven chemotherapeutic agents such as Rituxan.

**Example 6**

**IDEC-152 induces apoptosis in CD23+ tumor cells**

Having shown that the present invention may be used to effectively mediate ADCC activity and lyse tumor cells, anti-CD23 antibodies were examined to determine to what extent they could be used to induce apoptosis in malignant cells. In this regard, Table 3 immediately below, shows apoptosis measured by a caspase-3 activation assay substantially as set forth in Example 1. Percent apoptosis was documented at 4 and 24 hours using mean fluorescent intensity in log scale (MFI).

**Table 3**

| Caspase-3 activation by IDEC-152 (p5E8) in SKW cells | | |
| --- | --- | --- |
| **Culture Condition** | **% Apoptosis (MFI)[a]** | |
| | **4 hours** | **24 hours** |
| **SKW cells** | | |
| Cells only | 4.00 (2.16) | 4.73 (12.73) |
| Cells + IDEC-152 (p5E8) | 3.80 (15.65) | 3.65 (11.17) |
| Cells + IDEC-152 (p5E8) + anti-hu.IgG.F(ab')$_2$ | **80.26 (18.85)** | **60.51 (20.45)** |
| Cells + Rituxan (C2B8) | 4.12 (11.32) | 4.08 (20.57) |
| Cells + Rituxan (C2B8) + anti-hu.IgG.F(ab')$_2$ | **78.50 (24.10)** | **66.49 (25.0)** |
| Cells + CE9.1 | 4.34 (10.84) | 5.79 (12.40) |
| Cells + CE9.1 + anti-hu.IgG.F(ab')$_2$ | 7.57 (11.15) | 4.91 (13.42) |
| Cells + anti-hu.IgG.F(ab')$_2$ | 8.01 (11.86) | 4.12 (10.09) |
| [a] % Positive cells with caspase-3 activity and it's mean fluorescent intensity in log scale | | |

As seen in Table 3 above, SKW cells grown in the presence of IDEC-152 (p5E8γ1) did not show substantial activation of caspase-3. However cross-linking of IDEC-152 and Rituxan on the SKW cell surface resulted in increased activation of caspase-3. By comparison, cultures added with the isotype matched control antibody (CE9.1) of irrelevant specificity did not show any apoptosis. This confirms earlier results showing that the antibodies of the present invention may be used to induce apoptosis in tumor cells.

**Example 7**

**Fc receptors on effector cells can induce cross-linking of antibodies**

As noted above, cross-linking of the antibodies of the present invention enhances their ability to induce apoptosis in tumor cells. One mechanism for inducing apoptosis *in vivo* could be mediated via the Fc receptors on various effector cells. Accordingly, in this Example cells expressing Fc receptors were used to cross-link IDEC-152 and enhance the induction of apoptosis *in vitro*.

Briefly, SKW cells at $1 \times 10^6$ cells/ml density in culture media (RPMI-2% FCS) were incubated with 10μg/ml of IDEC-152 (p5E8) or Rituxan (C2B8) antibodies at 4°C in cell culture tubes. After 1 hour of incubation, the unbound antibody in the media was removed by centrifugation. The cells resuspended in growth media in appropriate volumes and added into 24 well tissue culture plates ($2 \times 10^6$ cells/well) seeded overnight with human Fc receptor (FcγRI) expressing CHO cells ($1 \times 10^5$) and incubated in 5%$CO_2$ at 37°C. Following incubation at different time points, cells were harvested and analyzed for apoptosis by flowcytometry based Tunel assay (BD Pharmingen, Cat # 6536 KK). It will be appreciated that the Tunel assay measures DNA fragmentation, an event that occurs during the late stages of apoptotic death. Flowcytometric analysis was performed on Becton-Dickinson FACScan using a FACScan Research Software package and the final data analysis was performed using the WinList Software package (Variety Software House). Percentage of cells positive for apoptosis was determined as the percentage of gated cells that were positive above the background, autofluorescence. Cells incubated with RF2 (IgG1) served as the negative controls for the experiment. The results of these measurements is shown immediately below in Table 4.

**Table 4**

| Cross-linking of IDEC-152 and C2B8 on via FcγRI leads to apoptosis | |
|---|---|
| **Antibody** | **% Apoptosis** |
| IDEC-152 (p5E8) | 56.31 |
| Rituxan (C2B8) | 56.07 |
| RF2 | 36.88 |
| No Antibody | 6.06 |

The results presented above indicate that cross-linking of IDEC-152 and Rituxan via FcγRI triggered SKW cells to undergo apoptosis. This Example serves to demonstrate that naturally occurring receptors on the surface of various effector cells can lead to antibody cross-linking and subsequent apoptosis of CD23[+] malignant cells *in vivo*.

**Example 8**

**Induction of apoptosis by IDEC-152 and Rituxan in CD23[+] cells**

The ability of IDEC-152 to induce apoptosis in CD23[+] malignant B cells was further shown *in vitro* using SKW lymphoma cells. Apoptosis was detected by a caspase-3 assay substantially as set forth in Example 1. For this Example, SKW cells at $0.5 \times 10^6$ cells/ml density in culture media (RPMI-2% FBS) were incubated with increasing doses of IDEC-152 or Rituxan antibodies at 4°C in cell culture tubes on ice. After 1 hour of incubation the unbound antibody in the media was removed by centrifugation. The cells were resuspended in growth media in appropriate volumes and added into 24 well tissue culture plates ($1.5 \times 10^6$ cells/well) with and without the addition of goat anti-human IgG specific secondary antibody (15μg/ml for cross-linking). Following incubation for 18 hours, cells were harvested and analyzed for apoptosis by flowcytometry substantially as described in Example 1.

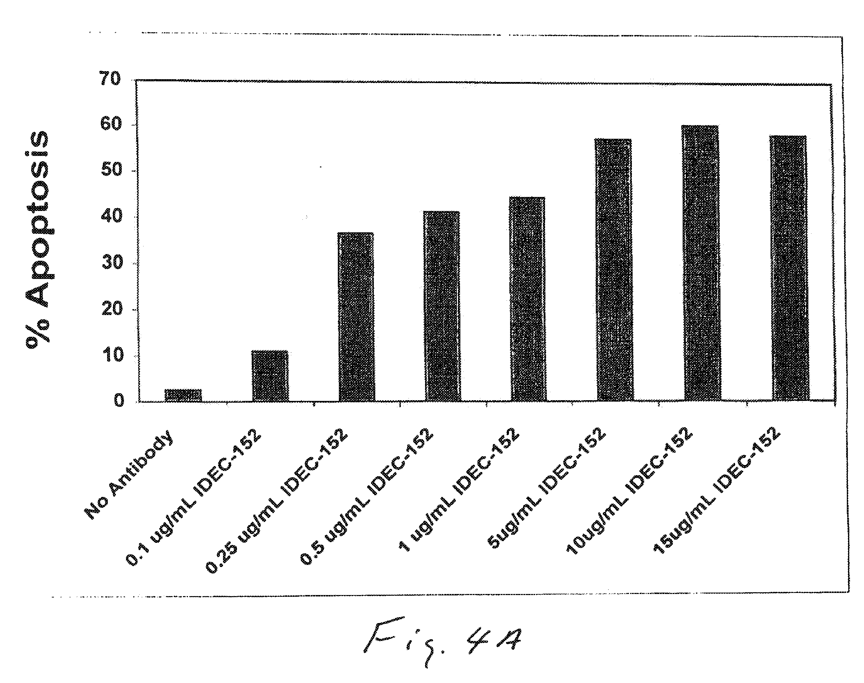

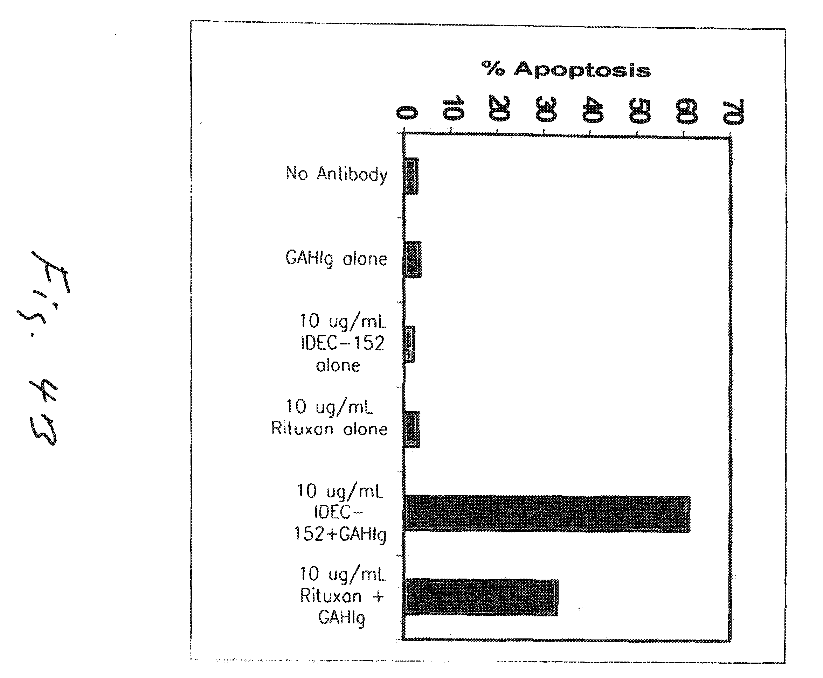

Figs. 4A and 4B illustrate that anti-CD23 antibodies may be used to effectively induce apoptosis in CD23[+] tumor cells. More specifically, Fig. 4A shows that increasing concentrations of cross-linked IDEC-152 result in increased apoptosis in SKW cells. At concentrations of 5 μg/mL of IDEC-152 and higher, approximately 60% of the cells underwent apoptosis during the incubation period. Similarly, Fig. 4B serves to illustrate that cross-linking antibodies to both CD20 and CD23 can substantially increase the rate of apoptosis induction in tumor cells. These data provide further evidence for a novel mechanism by which the instant invention can serve to eliminate tumor cells from a patient in need thereof.

**Example 9**

**IDEC-152 induced apoptosis in CD23+ cells at different time points**

To further elucidate mechanisms associated with the present invention the progress of apoptosis was measured in SKW cells at different time points.

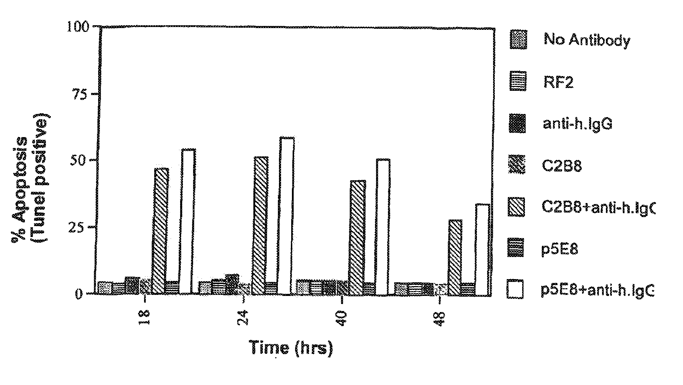

SKW cells at $1 \times 10^6$ cells/ml density in culture media (RPMI-2% FBS) were incubated with 10 μg/ml of p5E8 (IDEC-152) or C2B8 (Rituxan) antibodies at 4°C in tell culture tubes. After 1 hour of incubation the unbound antibody in the media was removed by centrifugation. The cells were resuspended in growth media in appropriate volumes and added into 24 well tissue culture plates ($2 \times 10^6$ cells/well) with and without the addition of goat anti-human IgG specific secondary antibody (50μg/ml) to provide cross-linking. Following incubation at different time points, cells were harvested and analyzed for apoptosis by flowcytometry based Tunel assay described in Example 7. The results of this assay are graphically illustrated in Fig. 5.

As with the earlier Examples set forth herein, Fig. 5 shows that the cross-linking of p5E8 (IDEC-152) and Rituxan with a secondary anti-Ig γ-specific antibody substantially enhanced apoptosis of CD23+ SKW cells. Interestingly, while the extent of apoptosis appears to drop off over time, it remains significant for a period of two full days. As expected, substantial apoptosis was not observed in cells incubated with RF2 and the secondary antibody or the secondary antibody alone.

**Example 10**

**IDEC-152 synergizes with Rituxan to induce apoptosis in CD23+ cells**

Additional unexpected advantages of the present invention include the ability of anti-CD23 antibodies to enhance the effectiveness of various chemotherapeutic agents including biologics such as Rituxan. This Example serves to illustrate such advantages.

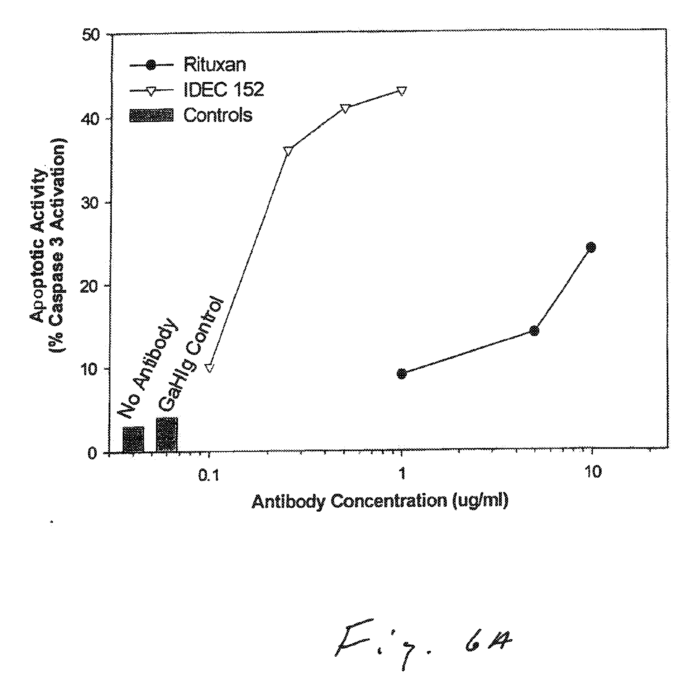

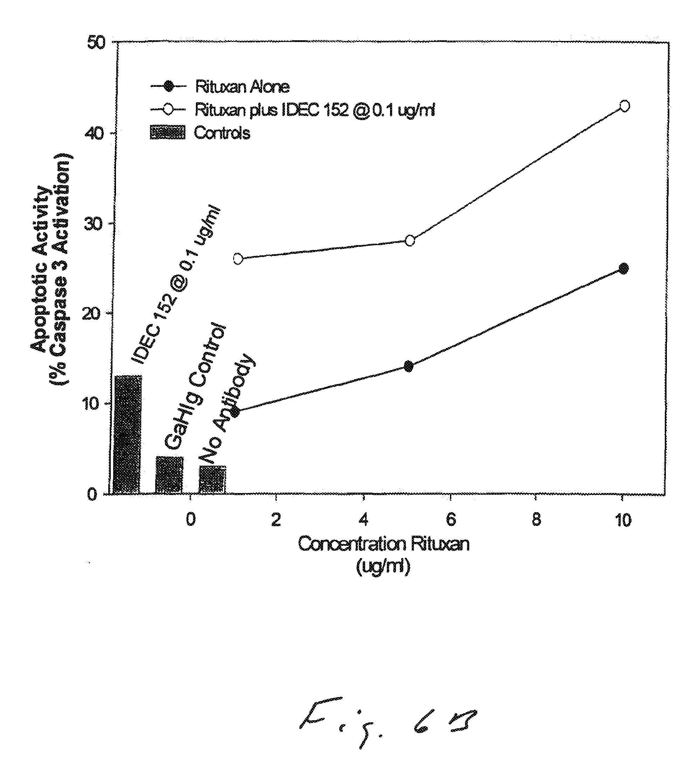

More particularly, this Example shows the apoptotic effects of increasing concentrations of an anti-CD23 antibody on SKW cells both alone and in combination with Rituxan. Using the caspase-3 assay substantially as described in Example 8, cross-linked anti-CD23 antibody and Rituxan were incubated with SKW cells. In a first experiment, concentrations of both IDEC-152 and Rituxan were increased and the apoptotic activity of each individual antibody was determined. In a second experiment a fixed concentration of IDEC-152 was combined with various concentrations of Ritxuan to elucidate any synergistic effects. The experiments are shown in Figs. 6A and 6B respectively.

A review of Figs. 6A and 6B show that both IDEC-152 and Rituxan induced apoptosis in SKW cells after cross-linking with goat F(ab')$_2$ anti-human IgG (GaHIg). Specifically Fig. 6A shows that IDEC-152 induces between 40% and 50% apoptosis at levels of approximately 1 μg/ml while Rituxan exhibits somewhat less activity. In addition to the activity of the individual antibodies, Fig. 6B shows that the addition of increasing amounts of Rituxan to a fixed concentration of IDEC-152 (0.1 μg/ml) enhances apoptotic activity above either of the antibodies individually. In this respect, the addition of Rituxan to IDEC-152 at concentrations of 10μg/ml provides apoptotic rates of approximately 45%. This observed synergistic effect dramatically underscores the advantages of the instant invention.

**Example 11**

**IDEC-152 enhanced Rituxan - mediated apoptosis in CD23+ cells**

An additional experiment was performed to confirm the synergistic effects seen in Example 10 with respect to the apoptosis of SKW cells as derived from the combination of an anti-CD23 antibody and an anti-CD20 antibody.

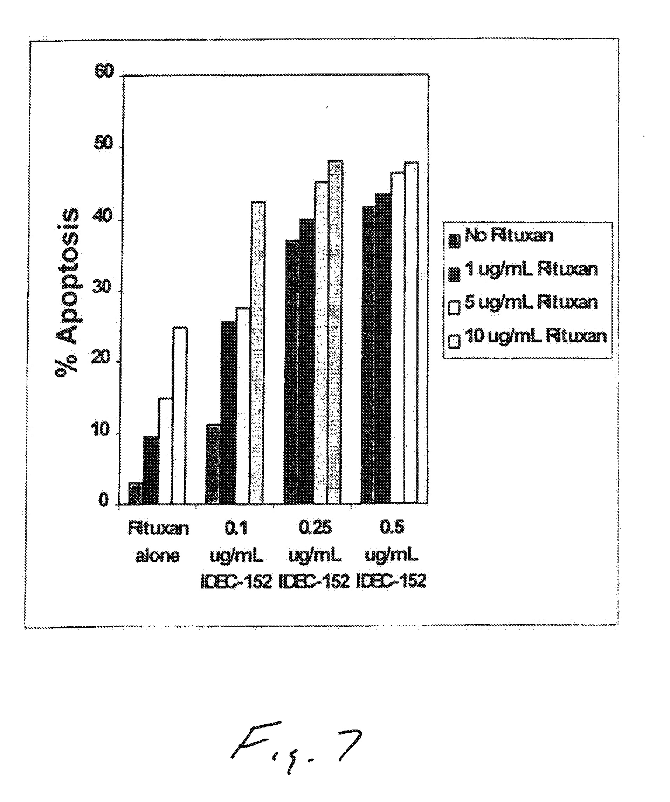

In this Example, SKW cells at a density of $0.5 \times 10^6$/mL were incubated on ice with increasing concentrations of IDEC-152, Rituxan or a combination of both. Following an hour, cells were pelleted down and resuspended in 2% FCS RPM1 and 15 ug/mL goat F(ab')$_2$ anti-human IgG for cross-linking. After 18 hours incubation at 37°C, apoptosis was measured by caspase-3 assay as described in Example 1. The results are shown in Fig. 7.

Fig. 7 unambiguously illustrates that the combination of an anti-CD23 antibody such as IDEC-152 with an anti-CD20 antibody such as Rituxan provides for enhanced apoptosis in malignant cell lines. Even at relatively low concentrations of IDEC-152 (i.e. 0.1 μg/ml), the apoptotic rate was approximately twice that of cells incubated with Rituxan alone. Fig. 7 further shows that this effect was enhanced at higher concentrations of IDEC-152.

**Example 12**

**IDEC-152 synergizes with Adriamycin in inducing apoptosis in CD23⁺ cells**

To demonstrate the versatility and wide applicability of the present invention, experiments were performed to show that the methods of the present invention are compatible with a number of chemotherapeutic agents. More particularly, the instant example demonstrates that anti-CD23 antibodies could be used effectively to enhance the efficacy of clinically approved chemotherapeutic agents (here Adriamycin).

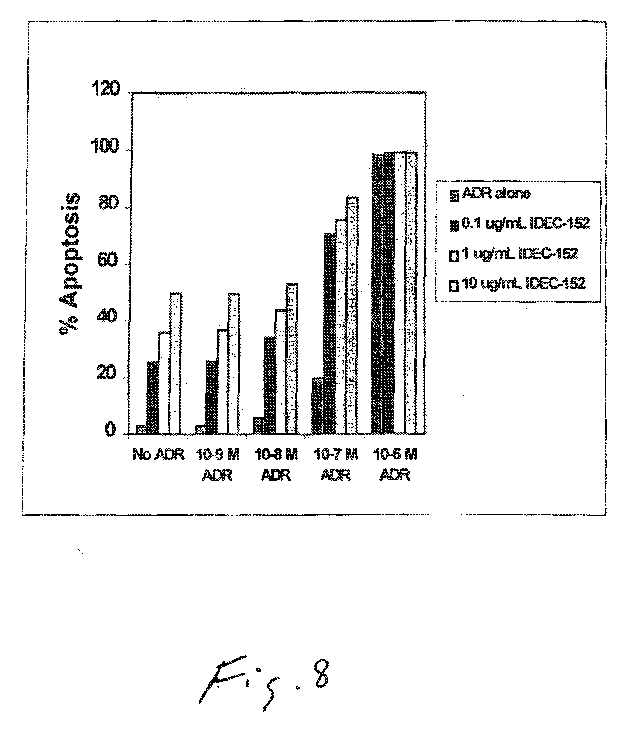

This experiment was performed using substantially the same procedure as set forth in Example 11 except that Adriamycin was used in combination with IDEC-152 rather than Rituxan. Prescription grade Adriamycin RDF (doxorubicin hydrochloride - NDC 0013-1086-91) was obtained from Pharmacia and Upjohn. Various concentrations of Adriamycin were combined with three different concentrations of IDEC-152 and the resulting rate of apoptosis in SKW cells was measured using the flow-cytometry based caspase 3 assay as described in Example 1. The results are shown in Fig. 8.

Fig. 8 graphically shows that the addition of IDEC-152 substantially increases the apoptotic effectiveness of Adriamycin at all concentrations charted. These synergistic effects are dramatically illustrated at the relatively low concentration of Adriamycin at $10^{-7}$ M where the addition of as little as 0.1 $\mu$g/ml of IDEC-152 increases the percentage of cellular apoptosis to approximately 70% versus less than 20% when no IDEC-152 is present. Those skilled in the art will appreciate that this is a significant improvement and would likely be reflected in clinical efficacy.

**Example 13**

**IDEC-152 synergizes with fludarabine in inducing apoptosis in CD23⁺ cells**

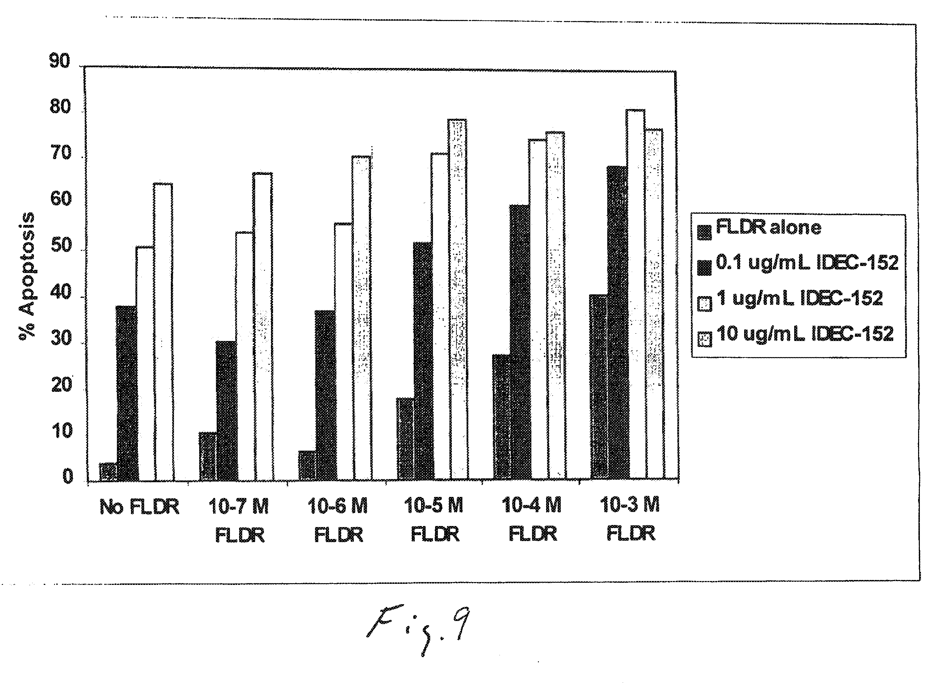

In another demonstration of the versatility of the present invention, the experiment set forth in Example 12 was repeated with the widely used chemotherapeutic agent fludarabine in place of Adriamycin. Prescription grade Fludara (fludarabine phosphate - NDC 504-19-511-06) was obtained from Berlex Corporation. The results were obtained and charted in Fig. 9 substantial as set forth in Example 12.

A review of Fig. 9 clearly indicates that the methods and compositions of the present invention may be used to substantially increase the rate of apoptosis in SKW cells when compared with fludarabine alone. In this respect, the addition of as little as 0.1 $\mu$g/ml IDEC-152 to solutions of $10^{-5}$ M fludarabine to increases the rate of apoptosis from less than 20% to greater than 50%. As with Example 12, this Example clearly validates the effectiveness of the present invention with clinically useful chemotherapeutic agents.

**Example 14**

**Anti-CD23 antibodies induce apoptosis in B-CLL cells**

As set forth herein the methods and compositions of the present invention are applicable to a wide range of malignancies including, in preferred embodiments, CLL. To directly demonstrate the effectiveness of the instant invention in the treatment of CLL, the ability of an anti-CD23 antibody to induce apoptosis in such cells was tested.

Peripheral blood monocytes (PBMC) were isolated from blood of CLL patient donors by Ficoll gradient by standard methods. Cell viability was determined using trypan blue exclusion assay to be close to 100% and all experiments were set up with fresh CLL cells. The cells were phenotyped for CD19, CD20 and CD23 expression by flow cytometry. Leukemia cells from CLL patients ($0.5-1 \times 10^6$ cells/ml) were incubated with p5E8 (10ug/ml) or control antibody (CE9.1, anti-CD4 antibody) on ice. After 1 hour of incubation, cells were spun down to remove unbound antibodies and resuspended at $1 \times 10^6$ cells/ml in growth medium (5% FCS-RPMI) and cultured in tissue culture tubes. The cells surface bound antibodies were cross-linked by spiking $F(ab')_2$ fragments of goat anti-human Ig-Fc$\gamma$ specific antibodies at 15$\mu$g/ml and the cultures were incubated at 37°C until assayed for apoptosis. In this regard, Table 5 immediately below, shows apoptosis measured by a caspase-3 activation assay substantially as set forth in Example 1. Percent apoptosis was documented at 4 and 24 hours using mean fluorescent intensity in log scale (MFI).

**Table 5**

| Caspase-3 activation by IDEC-152 (p5E8) on B-CLL cells from CLL patients | | |
|---|---|---|
| Culture Condition | % Apoptosis (MFI) | |
| | 4 hours | 24 hours |
| **CLL cells** | | |
| Cells only | 4.36 (14.34) | 5.08 (17.62) |
| Cells + IDEC-152 (p5E8) | 17.67 (10.66) | 20.08 (15.92) |
| Cells + IDEC-152 (p5E8) + anti-hu.IgG.F(ab')$_2$ | **54.82 (22.80)** | **35.63 (26.84)** |
| Cells + anti-hu.IgG.F(ab')$_2$ | 16.09 (12.27) | 20.85 (17.27) |

This Example unequivocally shows that the methods and compositions of the instant invention are effective in triggering programmed cell death in leukemia based neoplasms.

**Example 15**

**Induction of apoptosis by IDEC-152 and Rituxan in CLL cells**

Having shown the ability of CD23 antagonists such as IDEC-152 to induce apoptosis in CLL cells, additional experiments were performed to determine the efficacy of the antagonist by itself and in combination with a biologic chemotherapeutic agent (i.e. Rituxan).

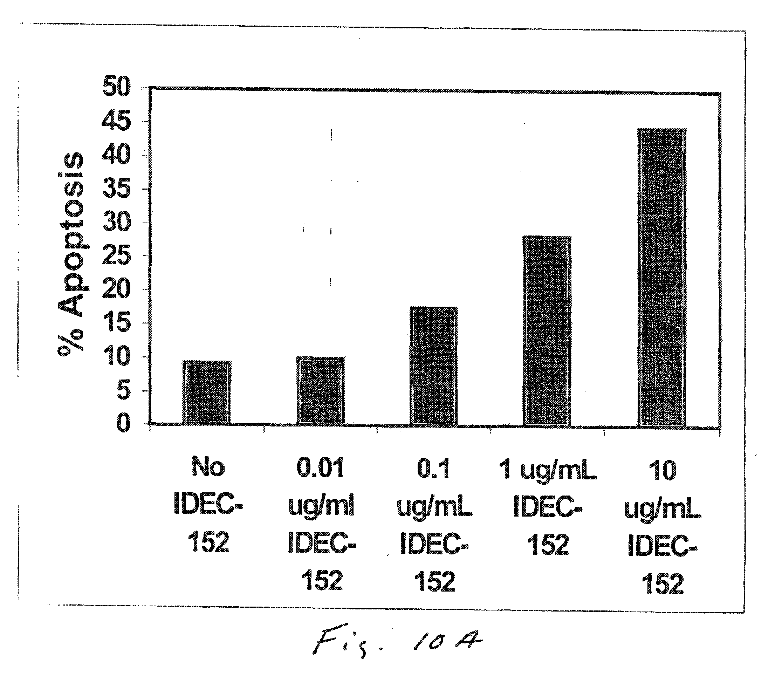

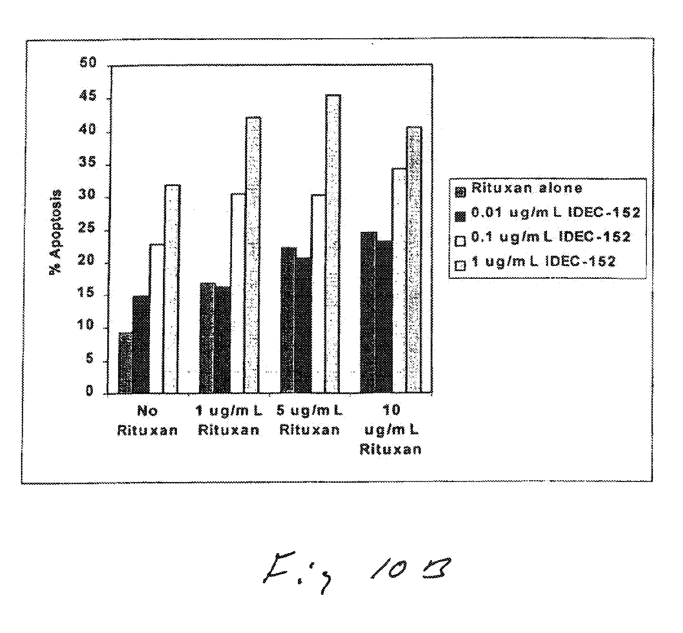

As with Example 14, Leukemia cells from CLL patients (1x10$^6$ cells/ml) were phenotyped and incubated with various concentrations of IDEC-152 or IDEC-152 and Rituxan on ice. After 1 hour of incubation, cells were spun down to remove unbound antibodies and resuspended in growth medium (2% FCS-RPMI) and cultured 24 well plates. The cells surface bound antibodies were cross-linked by spiking F(ab')$_2$ fragments of goat anti-human Ig-Fc$\gamma$ specific antibodies at 15 $\mu$g/ml and the cultures were incubated at 37°C for 18 hours when they were assayed for apoptosis using the caspase assay described in Example 1. The results are shown in Figs. 10A and 10B.

A review of Fig. 10A confirms the results seen in Example 14 in that CD23 antagonists such as IDEC-152 may be used to induce apoptosis in leukemia cells. At 1 $\mu$g/ml IDEC-152 had effectively induced apoptosis in approximately 30% of the CLL cells. Fig. 10B shows that, while IDEC-152 can induce apoptosis on its own in CLL cells, this effect may be enhanced through the addition of Rituxan. More specifically, Fig. 10B shows that the addition of varying concentrations of Rituxan substantially increases the percentage of cells undergoing apoptosis at the three concentrations of IDEC-152 tested. This observed synergy further accentuates the potential clinical efficacy of the present invention.

**Example 16**

**Induction of apoptosis by IDEC-152 and fludarabine in CLL cells**

In another demonstration of the usefulness of the present invention, CD23 antagonists were used in combination with the common chemotherapeutic agent fludarabine to induce apoptosis in CLL cells.

Purified B-cells from CLL patients were obtained and processed as previously described. Prescription grade Fludara (fludarabine phosphate - NDC 504-19-511-06) was obtained from Berlex Corporation. Cells were either treated with IDEC-152 alone, fludarabine alone or a combination of the two at various doses substantially as described in Example 15. Percent apoptosis was detected by a flowcytometry based caspase 3 assay as described in Example 1.

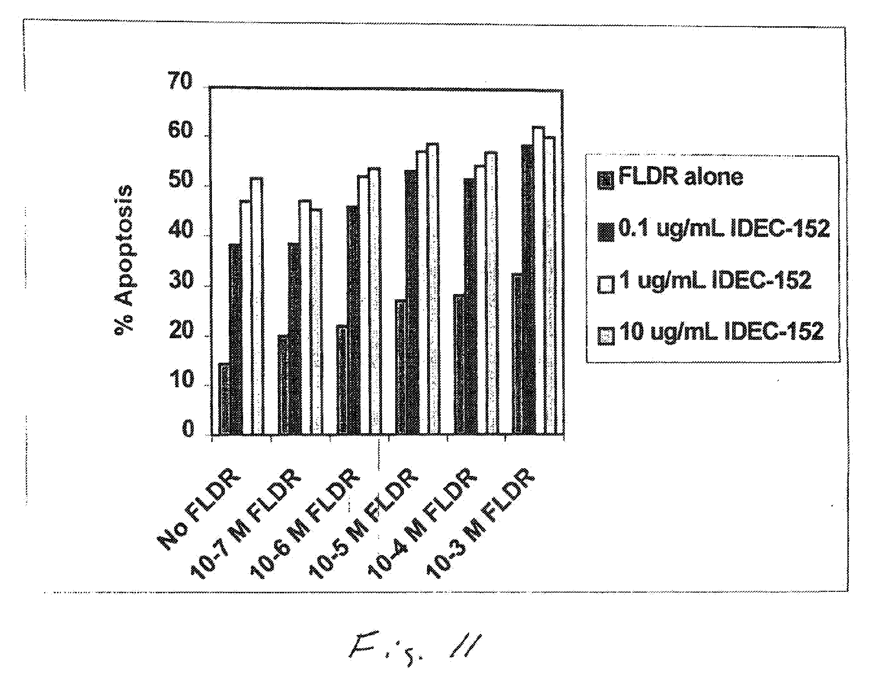

The results of this experiment, represented in Fig. 11, indicate that while both IDEC-152 and fludarabine exhibited some dose-dependent induction of apoptosis, a combination of the two compounds dramatically enhanced the rate of programmed cell death. These data indicate that IDEC-152, alone or in combinationj with other agents, might be effective in treatment of patients that may have become refractory to fludarabine or other chemotherapeutics.

**Example 17**

**Anti-tumor activity of IDEC-t52 *in vivo***

After demonstrating that the CD23 antagonists of the present invention are effective in mediating ADCC and apoptotic activity in various neoplastic cells *in vitro*, experiments were performed to show that the antagonists could kill malignant *cells in vivo*. More particularly, since the CD23 antigen is expressed at high density in human CLL patients, and often expressed at various antigen densities in patients with B-cell NHL, it was of interest to determine whether CD23 antagonists, either alone or in combination with chemotherapeutic agents, could mediate an anti-tumor response in an animal model.

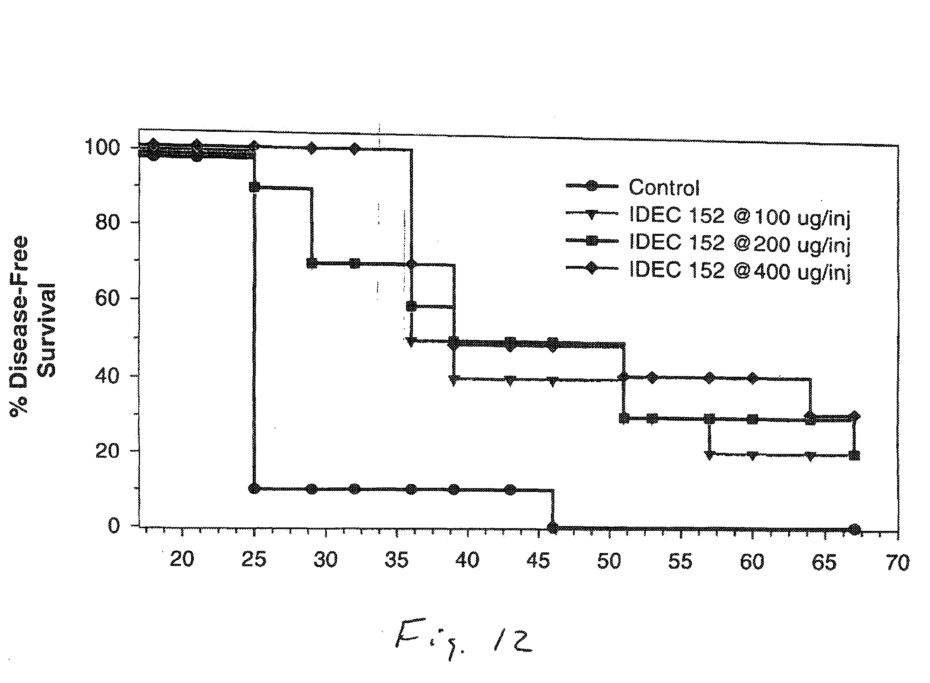

IDEC 152 was tested for anti-tumor activity in a human B-lymphoma/SCID mouse model that is commonly used in the art and predictive of clinical success. Animals were injected intravenously with SKW cells (CD20$^+$, CD23$^+$). SKW cells (4 x 10$^6$ viable in 100 $\mu$l HBSS buffer) were injected (*iv*) into the tail veins of 6-8 week old female CB17 SCID mice. One day after tumor inoculation, mice were injected (*ip*) with IDEC 152 in 200 $\mu$l HBSS buffer. Treatment was repeated every 2 days for a total of 6 MAb injections (Q2dx6). There were 10 animals used for each treatment and control (untreated, injected with 200 ul HBSS buffer) group. Animals were observed for signs of disease and survival monitored. All mice showing signs of disease developed a paralytic form before death. Mice that died between observation periods or mice that developed severe paralysis in both legs accompanied by labored breathing were sacrificed and scored as dead. Kaplan-Meier analysis was performed using the Statistical Analysis System (SAS) and *p*-values were generated by the Log-rank test. The results are shown in Fig. 12.

Fig. 12 clearly shows that the CD23 antagonists of the instant invention retarded the growth of tumors in the mice and led to a dramatic increase in survival when compared with the untreated controls. Specifically, anti-tumor activity was evidenced by increased survival of tumor bearing animals over non-treated controls at all doses tested (100, 200 and 400 $\mu$g antibody per injection). At the two higher doses 50% of the animals in the treated groups were still alive at day 46 when <u>all</u> of the control animals were dead. Significantly, 30% of the treated animals in these groups were still alive when the experiment concluded twenty days after the last control animal had died (i.e. day 66). These results are significant evidence as to the efficacy of the compounds of the instant invention when used alone.

## Example 18

### IDEC-152 synergizes with Rituxan to induce anti-tumor activity

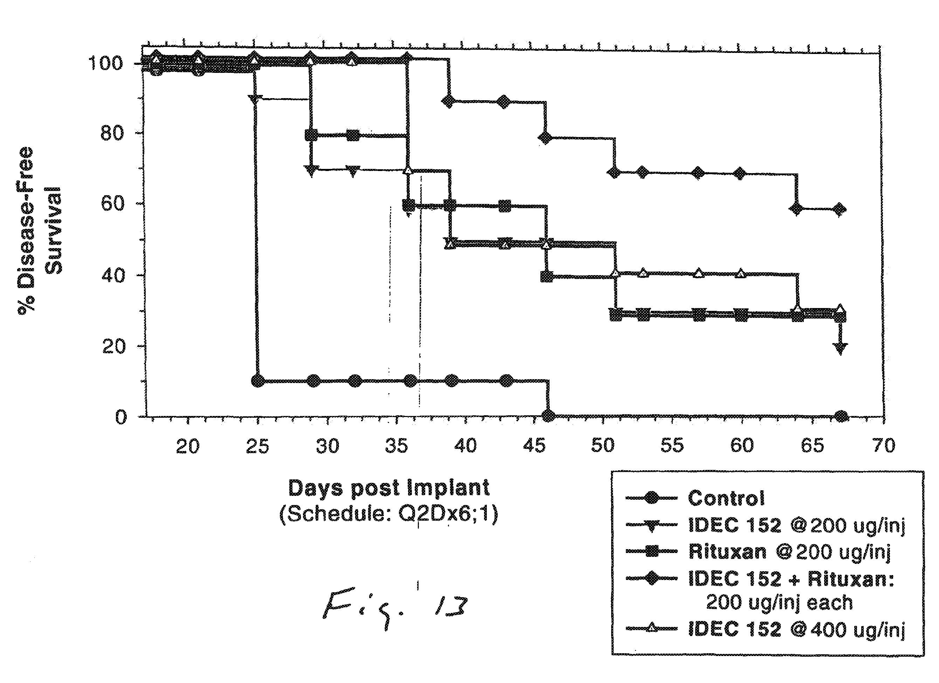

Having demonstrated that the CD23 antagonists were extremely effective tumorcidal agents when used alone, experiments were performed to explore the effectiveness of such compounds in concert with proven chemotherapeutic agents. To that end, the CD23 antagonists of the instant invention were tested in combination with Rituxan using the SKW/SCID mouse model as described in Example 17. For this experiment the mice were injected (*ip*) either with IDEC 152, Rituxan, or IDEC 152 plus Rituxan in 200 ul HBSS buffer at predetermined times after tumor inoculation. The results of the experiment are shown in Fig. 13.

Fig. 13 shows that the anti-tumor activity of IDEC 152 plus Rituxan was greater than the anti-tumor activity of each antibody tested alone (p $\leq$ 0.01). Using the same dosing schedule, the combination of IDEC 152 and Rituxan was clearly superior to the anti-tumor activity of each individual monoclonal antibody. This was evidenced by the fact that at day 68 there was a 60% survival rate of the animals in the combination antibody treatment group, compared to a 20% survival in the IDEC 152 group and a 30% survival in the Rituxan group. Significantly 6 out of 10 animals in the combination group were disease-free on day 68, more than twenty days after the last untreated animal had died. Furthermore, a greater tumor response was observed in mice receiving 200 ug per injection of IDEC152/Rituxan than mice receiving 400 ug per injection IDEC 152 alone, suggesting a synergistic response of combination therapy. Overall these results by day 46 clearly indicated that the combination of CD23 antagonists plus Rituxan could provide a synergistic anti-tumor response against a human malignancies in a murine xenograft model of disseminated disease.

Those skilled in the art will further appreciate that the present invention may be embodied in other specific forms without departing from the spirit or central attributes thereof. In that the foregoing description of the present invention discloses only exemplary embodiments thereof, it is to be understood that other variations are contemplated as being within the scope of the present invention. Accordingly, the present invention is not limited to the particular embodiments that have been described in detail herein. Rather, reference should be made to the appended claims as indicative of the scope and content of the invention

Preferred embodiments of the present invention are described below and are referred to as embodiments E1 ot E54.

E1. A method of treating a neoplastic disorder in a mammal in need thereof comprising administering a therapeutically effective amount of a CD23 antagonist to said mammal.

E2. The method of E1 wherein said CD23 antagonist is selected from the group consisting of CD23 reactive polypeptides, CD23 reactive peptides, CD23 reactive small molecules, and combinations thereof.

E3. The method of E2 wherein said CD23 reactive polypeptide comprises a monoclonal antibody or a polyclonal antibody.

E4. The method of E3 wherein said CD23 reactive polypeptide comprises a monoclonal antibody.

E5. The method of E4 wherein said monoclonal antibody is selected from the group consisting of chimeric antibodies and humanized antibodies.

E6. The method of E5 wherein said monoclonal antibody is a chimeric antibody and said chimeric antibody is primatized.

E7. The method of E6 wherein said primatized antibody is IDEC-152.

E8. The method of E7 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS-related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E9. The method of E8 wherein said neoplastic disorder is B cell chronic lymphocytic leukemia (B-CLL).

E10. The method of E1 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS-related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E11. The method of E10 wherein said neoplastic disorder is B cell chronic lymphocytic leukemia (B-CLL).

E12. The method of E1 wherein said CD23 antagonist is associated with a cytotoxic agent.

E13. The method of E12 wherein said cytotoxic agent is a radioisotope.

E14. The method of E1 further comprising the step of administering a chemotherapeutic agent.

E15. The method of E14 wherein said chemotherapeutic agent comprises an antibody.

E16. The method of E15 wherein said antibody reacts with or binds to CD19, CD20, CD22, CD40, CD40L, CD52 or B7.

E17. The method of E14 wherein said chemotherapeutic agent comprises fludarabine.

E18. A method of treating a neoplastic disorder in a mammal comprising the steps of:

administering a therapeutically effective amount of at least one chemotherapeutic agent to said mammal; and administering a therapeutically effective amount of at least one CD23 antagonist to said patient wherein said chemotherapeutic agent and said CD23 antagonist may be administered in any order or concurrently.

E19. The method of E18 wherein said CD23 antagonist is selected from the group consisting of CD23 reactive polypeptides, CD23 reactive peptides, CD23 reactive small molecules, and combinations thereof.

E20. The method of E19 wherein said CD23 reactive polypeptide comprises a monoclonal antibody or a polyclonal antibody.

E21. The method of E20 wherein said CD23 reactive polypeptide comprises a monoclonal antibody.

E22. The method of E21 wherein said monoclonal antibody is selected from the group consisting of chimeric antibodies and humanized antibodies.

E23. The method of E22 wherein said monoclonal antibody is IDEC-152.

E24. The method of E18 wherein said chemotherapeutic agent comprises an antibody.

E25. The method of E24 wherein said antibody reacts with or binds to CD19, CD20, CD22, CD40, CD40L, CD52 or B7.

E26. The method of E18 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E27. The method of E18 wherein said neoplastic disorder is B cell chronic lymphocytic leukemia (B-CLL).

E28. A method of treating B cell chronic lymphocytic leukemia (B-CLL) in a mammal in need thereof comprising administering a therapeutically effective amount of a CD23 antagonist to said mammal.

E29. The method of E28 wherein said CD23 antagonist is selected from the group consisting of CD23 reactive polypeptides, CD23 reactive peptides, CD23 reactive small molecules, and combinations thereof.

E30. The method of E29 wherein said CD23 reactive polypeptide comprises a monoclonal antibody or a polyclonal antibody.

E31. The method of E30 wherein said CD23 reactive polypeptide comprises a monoclonal antibody.

E32. The method of E31 wherein said monoclonal antibody is selected from the group consisting of chimeric antibodies and humanized antibodies.

E33. The method of E32 wherein said monoclonal antibody is IDEC-152.

E34. The method of E28 further comprising the step of administering a chemotherapeutic agent.

E35. The method of E32 wherein said chemotherapeutic agent comprises an antibody.

E36. The method of E35 wherein said antibody reacts with or binds to CD19, CD20, CD22, CD40, CD40L, CD52 or B7.

E37. The method of E34 wherein said chemotherapeutic agent comprises fludarabine.

E38. A method of treating a neoplastic disorder in a mammal comprising the steps of:

administering a therapeutically effective amount of Rituxan to said mammal; and

administering a therapeutically effective amount of IDEC-152 to said mammal wherein said Rituxan and said IDEC-152 may be administered in any order or concurrently.

E39. The method of E38 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E40. The method of E38 wherein said neoplastic disorder is B cell chronic lymphocytic leukemia (B-CLL).

E41. A method of inducing apoptosis in malignant cells comprising contacting said malignant cells with an apoptosis inducing amount of a CD23 antagonist.

E42. The method of E41 wherein said CD23 antagonist is selected from the group consisting of CD23 reactive polypeptides, CD23 reactive peptides, CD23 reactive small molecules, and combinations thereof.

E43. The method of E41 wherein said CD23 reactive polypeptide comprises a monoclonal antibody or a polyclonal antibody.

E44. The method of E43 wherein said CD23 reactive polypeptide comprises a monoclonal antibody.

E45. The method of E44 wherein said monoclonal antibody is selected from the group consisting of chimeric antibodies and humanized antibodies.

E46. The method of E44 wherein said monoclonal antibody is IDEC-152.

E47. The method of E41 further comprising the step of contacting said malignant cells with a chemotherapeutic agent.

E48. The method of E47 wherein said chemotherapeutic agent comprises an antibody.

E49. The method of E48 wherein said antibody reacts with or binds to CD19, CD20, CD22, CD40, CD40L, CD52 or B7.

E50. The method of E41 wherein said malignant cells are contacted *in vivo*.

E51. A kit useful for the treatment of a mammal suffering from or predisposed to a neoplastic disorder comprising at least one container having a CD23 antagonist deposited therein and a label or an insert indicating that said CD23 antagonist may be used to treat said neoplastic disorder.

E52. The kit of E51 wherein said neoplastic disorder is B cell chronic lymphocytic leukemia (B-CLL).

E53. The kit of E51 wherein said CD23 antagonist is a monoclonal antibody.

E54. The kit of E53 wherein said monoclonal antibody is IDEC-152.

**Claims**

1. Use of a CD23 antagonist, wherein the antagonist is an anti-CD23 antibody that is a tetramer of two light chains and two heavy chains, or an immunoreactive fragment thereof, for the manufacture of a medicament for the treatment of a neoplastic disorder in a mammal.

2. Use of a CD23 antagonist, wherein the antagonist is an anti-CD23 antibody that is a tetramer of two light chains and two heavy chains, or an immunoreactive fragment thereof, for the manufacture of a medicament for simultaneous, separate or sequential use in combination with one or more chemotherapeutic agents for treating a neoplastic disorder in a mammal.

3. Use according to claim 2, wherein said one or more chemotherapeutic agents is selected from the group consisting of cyclophosphamide, fludarabine, and a combination of cyclophosphamide and fludarabine.

4. Use according to claim 2, wherein said one or more chemotherapeutic agents comprises an antibody.

5. Use according to claim 4, wherein said antibody reacts with or binds to CD19, CD20, CD22, CD40, CD40L, CD52, or B7.

6. Use according to claim 4 or 5, wherein said antibody reacts with or binds to CD20.

7. Use according to claim 6, wherein said antibody that reacts with or binds to CD20 is rituximab.

8. Use according to any of claims 4 to 7, further comprising one or more additional chemotherapeutic agents.

9. Use according to claim 8, wherein said one or more additional chemotherapeutic agents is selected from the group consisting of cyclophosphamide, fludarabine, and a combination of cyclophosphamide and fludarabine.

10. Use according to any of the preceding claims, wherein said anti-CD23 antibody is IDEC-152.

11. Use according to any of claims 1 to 10, wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B CLL), lymphoplasmacytoid lymphoma (LPL), mantle cell lymphoma (MCL), follicular lymphoma (FL), diffuse large cell lymphoma (DLCL), Burkitt's lymphoma (BL), AIDS related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

12. Use according to claim 11, wherein said neoplastic disorder is B cell chronic lymphocytic leukemia (B-CLL).

13. Use of a CD23 antagonist, wherein the antagonist is an anti-CD23 antibody that is a tetramer of two light chains and two heavy chains, or an immunoreactive fragment thereof, for the manufacture of a medicament for inducing apoptosis in CD23+ malignant cells.

14. Use of a CD23 antagonist, wherein the antagonist is an anti-CD23 antibody that is a tetramer of two light chains and two heavy chains, or an immunoreactive fragment thereof, for the manufacture of a medicament for simultaneous, separate or sequential use in combination with one or more chemotherapeutic agents for inducing apoptosis in CD23+ malignant cells.

15. Use according to claim 14, wherein the one or more chemotherapeutic agents is selected from the group consisting of cyclophosphamide, fludarabine, an antibody that reacts with or binds to CD20, a combination of cyclophosphamide and fludarabine, and a combination of cyclophosphamide, fludarabine and an antibody that reacts with or binds to CD20.

Fig. 1

Fig 2

Fig. 3A

Fig. 33

Fig. 4A

Fig. 43

Fig. 6A

Fig. 6B

Fig. 7

Fig. 8

*Fig. 9*

Fig. 10A

Fig 10B

Fig. 11

Fig. 12

**Fig. 13**

## EUROPEAN SEARCH REPORT

**Application Number**

EP 09 15 7006

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 00/44788 A (HARIHARAN KANDASAMY ;LABARRE MICHAEL J (US); HANNA NABIL (US); HUY) 3 August 2000 (2000-08-03)<br>claims<br>* page 3, line 20 - line 23; examples 8,10 * | 1-15 | INV.<br>A61K45/00<br>A61K45/06<br>A61K39/395<br>A61P35/00<br>A61P35/02 |
| A,D | US 601 138 A (IDEC PHARMA CORP; SEIKAGAKU CORP) 4 January 2000 (2000-01-04)<br>* the whole document * | 1-15 | ADD.<br>A61K31/70 |
| A | US 5 843 439 A (ANDERSON DARRELL R [US] ET AL) 1 December 1998 (1998-12-01)<br>*claims*<br>* column 4, line 24 - line 34 * | | |
| A | BUSKE C ET AL: "MONOCLONAL ANTIBODY THERAPY FOR B CELL NON-HODGKIN'S LYMPHOMAS: EMERGING CONCEPTS OF A TUMOUR-TARGETED STRATEGY"<br>EUROPEAN JOURNAL OF CANCER, PERGAMON PRESS, OXFORD, GB,<br>vol. 35, no. 4, 1999, pages 549-557,<br>XP001010018<br>ISSN: 0959-8049<br>* page 552, right-hand column, line 13 - page 553, right-hand column, line 11 * | 1-15 | |

**TECHNICAL FIELDS SEARCHED (IPC)**

A61K
A61P

-/--

The present search report has been drawn up for all claims

| Place of search | Date of completion of the search | Examiner |
|---|---|---|
| Munich | 28 April 2009 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 09 15 7006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DALLAIRE B K ET AL: "NEW ASPECTS OF TREATMENT OF B-CELL NON-HODGKIN'S LYMPHOMAS WITH MONOCLONAL ANTIBODIES. IDEC-C2B8 (RITUXIMAB): BIOLOGY AND PRECLINICAL STUDIES" JOURNAL OF MOLECULAR MEDICINE, SPRINGER VERLAG, DE, vol. 75, no. 7, 1997, pages B230-B231, XP000952701 ISSN: 0946-2716 * abstract * | 1-15 | |
| A | SILBER R ET AL: "CHEMOSENSITIVITY OF LYMPHOCYTES FROM PATIENTS WITH B-CELL CHRONIC LYMPHOCYTIC LEUKEMIA TO CHLORAMBUCIL, FLUDARABINE, AND CAMPTOTHECIN ANALOGS" BLOOD, W.B. SAUNDERS, PHILADELPHIA, VA, US, vol. 84, no. 10, 15 November 1994 (1994-11-15), pages 3440-3446, XP000196389 ISSN: 0006-4971 * abstract; figure 4 * | 1-15 | |
| A | OUAAZ FATEH ET AL: "Growth arrest and terminal differentiation of leukemic myelomonocytic cells induced through ligation of surface CD23 antigen." BLOOD, vol. 84, no. 9, 1994, pages 3095-3104, XP001079834 ISSN: 0006-4971 * abstract * * page 3097, left-hand column, line 13 - line 28; figure 2 * | 1-15 | TECHNICAL FIELDS SEARCHED (IPC) |

-/--

The present search report has been drawn up for all claims

| Place of search | Date of completion of the search | Examiner |
|---|---|---|
| Munich | 28 April 2009 | Böhmerova, Eva |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ...........................................................................................
& : member of the same patent family, corresponding
   document

EPO FORM 1503 03.82 (P04C01)

Europäisches
Patentamt
European
Patent Office
Office européen
des brevets

**EUROPEAN SEARCH REPORT**

Application Number

EP 09 15 7006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | PATHAN NUZHAT ET AL: "Induction of apoptosis by IDEC-152 (anti-CD23) in lymphoma cells." BLOOD, vol. 98, no. 11 Part 1, 16 November 2001 (2001-11-16), page 367a, XP001079733 43rd Annual Meeting of the American Society of Hematology, Part 1;Orlando, Florida, USA; December 07-11, 2001, November 16, 2001 ISSN: 0006-4971 * abstract * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 28 April 2009 | Böhmerova, Eva |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 15 7006

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2009

| Patent document cited in search report | | Publication date | Patent family member(s) | | Publication date |
|---|---|---|---|---|---|
| WO 0044788 | A | 03-08-2000 | AT | 403680 T | 15-08-2008 |
| | | | AU | 775577 B2 | 05-08-2004 |
| | | | AU | 3473100 A | 18-08-2000 |
| | | | CA | 2359994 A1 | 03-08-2000 |
| | | | DK | 1157042 T3 | 08-12-2008 |
| | | | EP | 1157042 A1 | 28-11-2001 |
| | | | EP | 1970385 A2 | 17-09-2008 |
| | | | ES | 2311452 T3 | 16-02-2009 |
| | | | JP | 2002536969 T | 05-11-2002 |
| | | | PT | 1157042 E | 18-11-2008 |
| | | | US | 2005158828 A1 | 21-07-2005 |
| | | | US | 6897044 B1 | 24-05-2005 |
| US 601138 | A | | NONE | | |
| US 5843439 | A | 01-12-1998 | AT | 139900 T | 15-07-1996 |
| | | | AT | 196606 T | 15-10-2000 |
| | | | AU | 688743 B2 | 19-03-1998 |
| | | | BG | 62386 B1 | 29-10-1999 |
| | | | BG | 99701 A | 29-02-1996 |
| | | | CA | 2149329 A1 | 26-05-1994 |
| | | | DE | 69303494 D1 | 08-08-1996 |
| | | | DE | 69303494 T2 | 16-01-1997 |
| | | | DE | 69329503 D1 | 02-11-2000 |
| | | | DE | 69329503 T2 | 03-05-2001 |
| | | | DE | 122004000036 I1 | 07-07-2005 |
| | | | DK | 0669836 T3 | 14-10-1996 |
| | | | DK | 0752248 T3 | 13-11-2000 |
| | | | EP | 0669836 A1 | 06-09-1995 |
| | | | ES | 2091684 T3 | 01-11-1996 |
| | | | ES | 2152483 T3 | 01-02-2001 |
| | | | FI | 952327 A | 10-07-1995 |
| | | | GR | 3020731 T3 | 30-11-1996 |
| | | | JP | 8503468 T | 16-04-1996 |
| | | | JP | 3095175 B2 | 03-10-2000 |
| | | | JP | 4091235 B2 | 28-05-2008 |
| | | | JP | 2001010974 A | 16-01-2001 |
| | | | JP | 4203080 B2 | 24-12-2008 |
| | | | JP | 2006262907 A | 05-10-2006 |
| | | | LU | 91089 A9 | 16-09-2004 |
| | | | LV | 11732 B | 20-10-1997 |
| | | | MD | 1367 B2 | 31-12-1999 |
| | | | NL | 300156 I1 | 01-09-2004 |
| | | | NO | 951903 A | 13-07-1995 |
| | | | NO | 2006011 I1 | 02-10-2006 |
| | | | NO | 326271 B1 | 27-10-2008 |

For more details about this annex : see Official Journal of the European Patent Office, No. 12/82

**ANNEX TO THE EUROPEAN SEARCH REPORT
ON EUROPEAN PATENT APPLICATION NO.**

EP 09 15 7006

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2009

| Patent document cited in search report | Publication date | Patent family member(s) | Publication date |
|---|---|---|---|
| US 5843439 A | | NZ 258392 A | 22-09-1997 |
| | | PL 309002 A1 | 18-09-1995 |
| | | PT 752248 E | 31-01-2001 |
| | | RO 118524 B1 | 30-06-2003 |
| | | RU 2139731 C1 | 20-10-1999 |
| | | WO 9411026 A2 | 26-05-1994 |
| | | US 6682734 B1 | 27-01-2004 |
| | | US 6399061 B1 | 04-06-2002 |
| | | US 5776456 A | 07-07-1998 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 09772938 B
- US 09855717 B
- US 09985646 B
- US PN6011138 A
- US 5500362 A
- US 5821337 A
- US 379072 A, Newman
- US 5658570 A
- EP 368684 B1
- US PN5969108 A
- US 6075181 A
- US 5939598 A
- US 5591669 A
- US 5589369 A
- US 5811524 A
- US 5693780 A
- US 5756096 A
- US 5585089 A
- US 5693761 A
- US 5693762 A
- US 5736137 A
- US 4831175 A, Gansow

- US 5099069 A
- US 5246692 A
- US 5286850 A
- US 5434287 A
- US 5124471 A, Gansow
- US 08475813 B
- US 08475815 B
- US 08478967 B
- US 5460785 A
- US 5843439 A
- US 5776456 A
- US 4665077 A
- US 4120649 A
- WO 9008187 A
- US 5114721 A, Cohen
- WO 9011294 A
- WO 9101133 A
- EP 340109 A
- WO 9704801 A
- US 3773919 A
- US SN09259337 A
- US SN09259338 A

**Non-patent literature cited in the description**

- **Young ; Percy et al.** *NIH Monograph,* 1981, vol. 57
- Cancer Facts and Figures. American Cancer Society Publication, 1988
- **Dighiero ; Travade et al.** *Blood,* 1991, vol. 78, 1901
- **Gale ; Foon.** *Ann. Intern. Med,* 1985, vol. 103, 101
- **Foon ; Todd.** *Blood,* 1986, vol. 1, 1
- **Spiegelberg.** *Adv. Immunol.,* 1984, vol. 35, 61
- **Kikulani ; Suemura et al.** *J. Exp. Med,* 1986, vol. 164, 1455
- **Delespesse ; Suter et al.** *Adv. Immunol.,* 1991, vol. 49, 149
- **Delespesse ; Sarfati et al.** *Immunol. Rev.,* 1992, vol. 125, 77
- **Sarfati ; Bron et al.** *Blood,* 1988, vol. 71, 94
- **Ravetch ; Kinet.** *Annu. Rev. Immunol,* 1991, vol. 9, 457-92
- **Clynes et al.** *PNAS (USA),* 1998, vol. 95, 652-656
- **Kohler et al.** *Nature,* 1975, vol. 256, 495

- **Coding.** Monoclonal Antibodies: Principles and Practice. Academic Press, 1986, 59-103
- **Marks et al.** *Bio/Technology,* 1992, vol. 10, 779-783
- **Newman.** *Biotechnology,* 1992, vol. 10, 1455-1460
- Current Protocols in Immunology. Green Publishing Associates and Wiley-Interscience, John Wiley and Sons, 1991
- **Morrison et al.** *Proc. Natl. Acad. Sci.,* 1984, vol. 81 (685), 1-5
- **Morrison et al.** *Adv. Immunol.,* 1988, vol. 44, 65-92
- **Verhoeyen et al.** *Science,* 1988, vol. 239, 1534-1536
- **Padlan.** *Molec. Immun.,* 1991, vol. 28, 489-498
- **Padlan.** *Molec. Immun.,* 1994, vol. 31, 169-217
- **Ridgway, A. A. G.** Mammalian Expression Vectors. Butterworths, 1988, 470-472
- **Stinchcomb et al.** *Nature,* 1979, vol. 282, 39
- **Kingsman et al.** *Gene,* 1979, vol. 7, 141

- **Tschemper et al.** *Gene,* 1980, vol. 10, 157
- **Jones.** *Genetics,* 1977, vol. 85, 12
- **Murray.** *J. Nuc. Med.,* 1985, vol. 26, 3328
- **Carraguillo et al.** *J. Nuc. Med.,* 1985, vol. 26, 67
- **Peirersz et al.** *Immunol. Cell Biol.,* 1987, vol. 65, 111-125
- **Reff et al.** *Blood,* 1994, vol. 83, 435-445
- **Arnold S. Freedman ; Lee M. Nadler et al.** Malignant Lymphomas, in HARRISON'S PRINCIPLES OF INTERNAL MEDICINE. 1994, 1774-1788
- **Bruce A Chabner et al.** Antineoplastic Agents, in GOODMAN & GILMAN'S THE PHARMACOLOGICAL BASIS OF THERAPEUTICS. 1996, 1233-1287
- **Offner et al.** *Science,* 1991, vol. 251, 430-432
- **Ianeway.** *Nature,* 1989, vol. 341, 482
- **Wilman.** prodrugs in Cancer Chemotherapy. *Biochemical Society Transactions,* 1986, vol. 14, 375-382
- Prodrugs: A Chemical Approach to Targeted Drug Delivery. **Stella et al.** Directed Drug Delivery. Humana Press, 1985, 247-267
- Remington's Pharmaceutical Sciences. 1980
- Remington 's Pharmaceutical Sciences. 1980
- **Gaidono et al.** "Lymphomas", IN CANCER: PRINCIPLES & PRACTICE OF ONCOLOGY. 1997, vol. 2, 2131-2145
- **Epstein et al.** *Cancer,* 1978, vol. 42, 2379